# EUROPEAN PATENT APPLICATION

(11) **EP 2 835 097 A1**
(43) Date of publication of application: **11.02.2015**
(21) Application number: 14002736.8
(22) Date of filing: 05.08.2014
(51) Int. Cl.: A61B 3/028, A61B 3/032, A61B 3/10

(54) **Ophthalmologic imaging apparatus**

(30) Priority: 08.08.2013 JP 2013165638
(71) Applicant: Kabushiki Kaisha TOPCON, Tokyo 174 (JP)
(72) Inventor: Stanga, Paulo E., Greater Manchester, M13 9WL (GB); Makoto, Fujino, Tokyo, 174-8580 (JP); Hisashi, Tsukada, Tokyo, 174-8580 (JP)
(74) Representative: Gagel, Roland

(57) **Abstract**

An optical system of an ophthalmologic imaging apparatus of an embodiment divides light from a light source into measuring light and reference light, interferes the measuring light having passed through an eye with the reference light, and detects interference light. A processor generates examination data indicating the status of the eye by processing the detection results of the interference light. An output part outputs the examination data generated by the processor. The optical system presents a target to the eye by guiding a light flux output from a flat panel display to the eye. The processor derives a visual acuity value of the eye based on the content of a response input by using a manipulator when the response of a subject for the presented target is input via the manipulator, generating the examination data including this visual acuity value.

## Description

### [TECHNICAL FIELD]

The present invention relates to an ophthalmologic imaging apparatus for forming an image of an eye by using optical coherence tomography (OCT).

### [BACKGROUND ART]

In recent years, OCT that forms images of the surface morphology and internal morphology of an object by using a light beam from a laser light source or the like has attracted attention. Unlike an X-ray CT apparatus, OCT is noninvasive to human bodies, and is therefore expected to be utilized in the medical field and biological field. For example, in the ophthalmology, apparatuses that form images of a fundus and a cornea are in a practical stage.

Patent document 1 discloses a device to which a method called Fourier domain OCT or frequency domain OCT is applied. This device irradiates a low coherence light beam to an object, superposes its reflected light and reference light to generate interference light, acquires the spectral intensity distribution of the interference light, and executes Fourier transform on it , thereby imaging the morphology in the depth direction (z-direction) of the object. Moreover, the device is provided with a Galvano mirror that deflects a light beam (measuring light) in one direction (x-direction) orthogonal to the z-direction, and thereby forms an image of a desired measurement target region of the object. An image formed by this device is a two-dimensional cross sectional image in the depth direction (the z-direction) along the scanning direction (x-direction) of the light beam. It should be noted that the technique in which a spectrometer is employed is also called Spectral domain.

Patent document 2 discloses a technique in which: measuring light is deflected in the horizontal direction (x-direction) and the vertical direction (y-direction) to form multiple two-dimensional cross sectional images along the horizontal direction; and three-dimensional cross sectional data of a measured range is acquired and imaged based on the multiple cross sectional images. As the three-dimensional imaging, for example, a method of arranging and displaying multiple cross sectional images along the vertical direction (referred to as stack data or the like), and a method of executing a rendering process on volume data (voxel data) based on the stack data to form a three-dimensional image are considered.

Patent documents 3 and 4 disclose other types of OCT devices. Patent document 3 describes an OCT device configured to: scan (sweep) the wavelength of light that is irradiated to an object; detect interference light obtained by superposing, on the reference light, the reflected light of the irradiated light with respective wavelengths to acquire spectral intensity distribution; and execute Fourier transform to image the morphology of the object. Such an OCT device is called a Swept Source type or the like. The Swept Source type is a kind of the Fourier Domain type.

Further, Patent document 4 describes an OCT device configured to: irradiate light having a predetermined beam diameter onto an object; and analyze the components of interference light obtained by superposing the reflected light of the irradiated light and reference light, thereby forming an image of a cross-section of the object orthogonal to the travelling direction of the light. Such an OCT device is called a full-field type, en-face type or the like.

Patent document 5 discloses a configuration obtained by applying OCT in the ophthalmic field. It should be noted that before the use of OCT, a retinal camera, a slit lamp microscope SLO (scanning laser ophthalmoscope), etc. have been widely used as devices for observing an (refer to, for example, Patent document 6, Patent document 7 and Patent document 8). The retinal camera is a device that photographs a fundus by irradiating illumination light onto an eye and detecting the reflected light from the fundus. The slit lamp microscope is a device that obtains an image of the cross-section of a cornea by cutting off a light section of the cornea using slit light. The SLO is a device that images morphology of a retinal surface by scanning a fundus with laser light and detecting its reflected light with a high-sensitive element such as a photo multiplier.

A device using OCT is advantageous compared with a retinal camera etc. with respect to the fact that it is capable of acquiring high-definition images, and is also capable of acquiring cross sectional images and three-dimensional images.

In this manner, because the device using OCT may be applied to the observation of various sites of an eye and high-resolution images may be obtained, it is applied to the diagnosis of various ophthalmologic diseases. For example, a device that provides diagnostic information for maculopathy and gaucoma by combining an OCT device and a subjective optometer is known (refer to Patent document 9).

### [PRIOR ART DOCUMENTS]

### [PATENT DOCUMENTS]

[Patent Document 1]
   Japanese Unexamined Patent Application No. Hei 11-325849
[Patent Document 2]
   Japanese Unexamined Patent Application No. 2002-139421
[Patent Document 3]
   Japanese Unexamined Patent Application No. 2007-24677
[Patent Document 4]
   Japanese Unexamined Patent Application Publication No. 2006-153838
[Patent Document 5]
   Japanese Unexamined Patent Application Publication No. 2008-73099
[Patent Document 6]
   Japanese Unexamined Patent Application Publication No. Hei 9-276232
[Patent Document 7]
   Japanese Unexamined Patent Application Publication No. 2008-259544
[Patent Document 8]
   Japanese Unexamined Patent Application Publication No. 2009-11381
[Patent Document 9]
   Japanese Unexamined Patent Application Publication No. 2011-515194

### [SUMMARY OF THE INVENTION]

### [PROBLEM THAT THE INVENTION IS TO SOLVE]

Frequent confirmation of disease conditions is preferable depending on types of ophthalmologic diseases. For example, for age-related macular degeneration, administration timing is preferably managed while frequently confirming the disease conditions as medication is carried out in accordance with the disease conditions. In addition, the management of minimum necessary administration of medical agents at appropriate timings is also desired from the standpoint of patients, taking into account the fact that medical agents are relatively expensive.

Conventionally, it has been necessary for patients to frequently visit the hospital in order to achieve such desirable management. However, management for a long period of time is required for diseases such as age-related macular degeneration and glaucoma. Unfortunately, frequent hospital visits for a long period of time obviously impose a heavy burden on patients.

The present invention has been created in light of the above-described problems, with the object of providing an ophthalmologic imaging apparatus capable of preferably conducting ophthalmologic examinations at home or the like.

### [MEANS FOR SOLVING THE PROBLEM]

In order to achieve the above-mentioned object, the invention of Claim 1 is an ophthalmologic imaging apparatus, comprising: an optical system configured to divide light from a light source into measuring light and reference light, interfere the measuring light having passed through an eye with the reference light, and detect interference light thereby acquired; a processor configured to generate examination data indicating the status of the eye by processing the detection results of the interference light via the optical system; an output part configured to output the examination data generated by the processor; a controller; and a manipulator, wherein the optical system, which comprises a flat panel display configured to display a target for an eye examination by being controlled by the controller, presents the target to the eye by guiding a light flux output from the flat panel display to the eye, and the processor derives a visual acuity value of the eye based on the content of a response input by using the manipulator when the response of a subject for the presented target is input via the manipulator, generating the examination data including this visual acuity value.

The invention of Claim 2 is the ophthalmologic imaging apparatus according to Claim 1, wherein the controller controls the flat panel display to display a fixation target for fixating the eye.

The invention of Claim 3 is the ophthalmologic imaging apparatus according to Claim 2, wherein the controller, while carrying out control for allowing the flat panel display to display the target, carries out control allowing the optical system to detect the interference light.

The invention of Claim 4 is the ophthalmologic imaging apparatus according to Claim 3, wherein the controller allows the target to be displayed as the fixation target.

The invention of Claim 5 is the ophthalmologic imaging apparatus according to Claim 3, wherein the controller carries out control for allowing the flat panel display to display the fixation target upon carrying out control for allowing the optical system to detect the interference light.

The invention of Claim 6 is the ophthalmologic imaging apparatus according to any one of Claims 1 to 5, comprising a static-state determining part configured to determine whether or not the eye substantially remains still based on the data optically acquired, wherein the controller carries out control for allowing the optical system to detect the interference light when the static-state determining part determines that the eye remains substantially still.

The invention of Claim 7 is the ophthalmologic imaging apparatus according to any one of Claims 1 to 6, wherein the light source outputs infrared light, the flat panel display outputs visible light, and the optical system, which comprises a dichroic mirror configured to combine an optical path of the measuring light based on the infrared light and an optical path of the visible light output from the flat panel display, guides the measuring light and the visible light to the eye via the dichroic mirror.

The invention of Claim 8 is the ophthalmologic imaging apparatus according to any one of Claims 1 to 7, wherein the output part comprises a first communication part that can communicate with a first computer placed in a medical institution via communication lines, and the controller controls the first communication part to transmit the examination data generated by the processor and identification information of the eye to the first computer.

The invention of Claim 9 is the ophthalmologic imaging apparatus according to Claim 8, wherein the first communication part can communicate with a second computer placed in a medical institution via communication lines, and the controller controls the first communication part to transmit report information to the second computer when the processor has not generated the examination data for a specific period of time.

The invention of Claim 10 is the ophthalmologic imaging apparatus according to any one of Claims 1 to 9, comprising a first input part configured to input information indicating whether the eye is a left eye or a right eye into the controller, wherein the controller controls the optical system and the processor based on the information input by the first input part.

The invention of Claim 11 is the ophthalmologic imaging apparatus according to Claim 10, wherein the first input part comprises a right/left determining part configured to determine whether the eye is a left eye or a right eye, inputting the determination results into the controller, and the controller controls the optical system and the processor based on the determination results input by the right/left determining part.

The invention of Claim 12 is the ophthalmologic imaging apparatus according to any one of Claims 1 to 11, wherein the storage stores authorized personal authentication information in advance regarding an authorized subject that is allowed to carry out an examination using this apparatus, and the ophthalmologic imaging apparatus comprises: a second input part configured to input personal authentication information into the controller; and an authentication processor configured to determine whether or not the personal authentication information input by the second input part coincides with the authorized personal authentication information, and wherein the controller inhibits the operations of the optical system and the processor when the authentication processor determines that the personal authentication information does not coincide with the authorized personal authentication information.

The invention of Claim 13 is the ophthalmologic imaging apparatus according to any one of Claims 1 to 12, wherein the optical system comprises a scanner configured to scan the eye with the measuring light by being controlled by the controller.

The invention of Claim 14 is the ophthalmologic imaging apparatus according to any one of Claims 1 to 13, wherein the optical system comprises a focus position changing part configured to change the focus position of the measuring light for the eye by being controlled by the controller.

The invention of Claim 15 is the ophthalmologic imaging apparatus according to Claim 14, wherein the focus position changing part comprises: a focusing lens provided in the optical system; and a first driver configured to move the focusing lens by being controlled by the controller.

The invention of Claim 16 is the ophthalmologic imaging apparatus according to Claim 15, wherein the focusing lens is an objective lens.

The invention of Claim 17 is the ophthalmologic imaging apparatus according to Claim 14, wherein the focus position changing part comprises: a diopter correction lens; and a second driver configured to insert or remove the diopter correction lens into or from an optical path of the optical system by being controlled by the controller.

The invention of Claim 18 is the ophthalmologic imaging apparatus according to any one of Claims 1 to 17, wherein the optical system comprises a maximum interference depth changing part configured to change, by being controlled by the controller, the maximum interference depth at which the interference intensity between the measuring light and the reference light becomes maximum.

The invention of Claim 19 is the ophthalmologic imaging apparatus according to Claim 18, wherein the optical system comprises: a beam splitter configured to divide the light from the light source into measuring light and reference light; a measurement arm configured to guide the measuring light to the eye and guide the returned light of the measuring light from the eye to the beam splitter; and a reference arm comprising a mirror configured to reflect the reference light toward the beam splitter, and wherein the optical system is configured to detect the interference light between the returned light of the measuring light and the reference light acquired via the beam splitter, the maximum interference depth changing part comprises the mirror and a third driver configured to move the mirror in the direction along the optical axis of the reference arm, and the controller moves the mirror by controlling the third driver.

The invention of Claim 20 is the ophthalmologic imaging apparatus according to any one of Claims 1 to 19, wherein the processor comprises a layer thickness information generating part configured to generate layer thickness information of a fundus based on the detection results of the interference light by the optical system, and the examination data includes the layer thickness information generated by the layer thickness information generating part.

The invention of Claim 21 is the ophthalmologic imaging apparatus according to any one of Claims 1 to 20, wherein every time examination data is generated by the processor, the controller associates this examination data with date and time information and stores it in the storage.

The invention of Claim 22 is the ophthalmologic imaging apparatus according to any one of Claims 1 to 21, comprising a monitoring processor configured to monitor the operational status of a specific site of the apparatus, wherein the output part comprises a second communication part that can communicate with a third computer via communication lines, and the controller controls the second communication part to transmit information indicating the occurrence of abnormalities to the third computer when the monitoring processor detects abnormalities in the operational status.

The invention of Claim 23 is the ophthalmologic imaging apparatus according to Claim 22, comprising an information output part configured to output visual information and/or audio information, wherein the controller controls the information output part to output report information indicating the occurrence of abnormalities when the monitoring processor detects abnormalities in the operational status.

### [EFFECT OF THE INVENTION]

According to the present invention, it is possible to preferably conduct ophthalmologic examinations at home or the like.

### [BRIEF DESCRIPTION OF THE DRAWINGS]

Fig. 1 is a schematic diagram showing an example of a configuration of an ophthalmologic imaging apparatus of an embodiment.
Fig. 2 is a schematic diagram showing an example of a configuration of an ophthalmologic imaging apparatus of an embodiment.
Fig. 3A is a flowchart showing an example of an operation of an ophthalmologic imaging apparatus of an embodiment.
Fig. 3B is a flowchart showing an example of an operation of an ophthalmologic imaging apparatus of an embodiment.

### [MODE FOR CARRYING OUT THE INVENTION]

Examples of embodiments of an ophthalmologic imaging apparatus according to the present invention will be described in detail with reference to the drawings. The ophthalmologic imaging apparatus according to the present invention forms fundus cross-sectional images using OCT. In the specifications, images to be acquired through OCT are sometimes collectively referred to as OCT images. In addition, measurement operations for creating OCT images are sometimes referred to as OCT measurement. Further, the descriptions of the documents cited in the specifications can be appropriately incorporated as the content of the following embodiments.

The case of an applying spectrum domain type OCT will be described in detail in the following embodiments; however, the configuration of the present invention may be applied to ophthalmologic imaging apparatuses to which other types of OCT are applied. In addition, apparatuses according to the embodiments may have imaging functions other than OCT. The functions to acquire anterior segment and/or fundus front images are exemplary of such additional photographing functions (for example, refer to Patent document 5.)

### [Configuration]

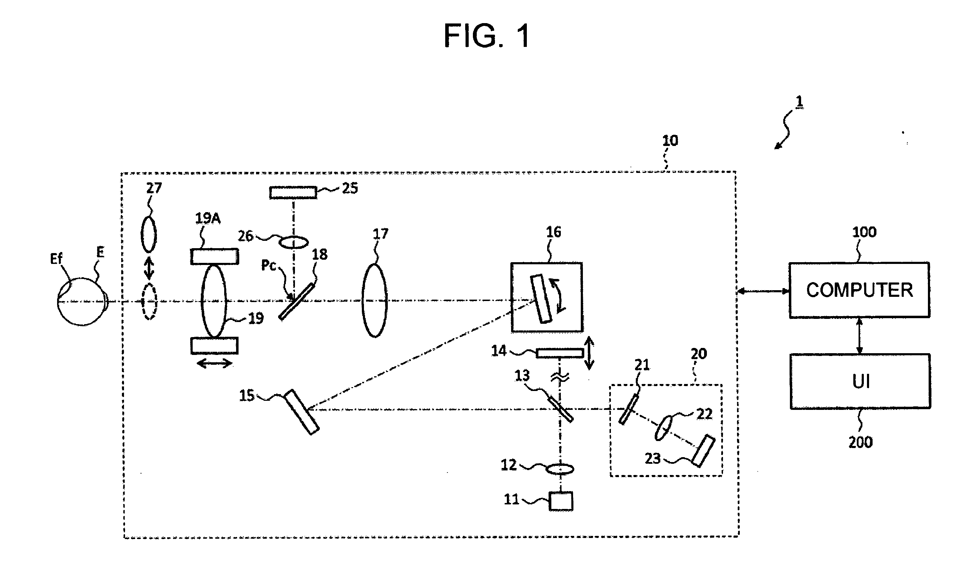

The configuration of the ophthalmologic imaging apparatus according to the embodiments will be described. An ophthalmologic imaging apparatus 1 illustrated in Fig. 1 includes an optical unit 10, a computer 100, and a user interface (UI) 200.

The optical unit 10, the computer 100, and the user interface 200 may be integrally provided (that is, provided in a single housing). Alternatively, they may be provided, in a decentralized fashion, in two or more housings. In this case, a part of the ophthalmologic imaging apparatus may be provided in another apparatus. For example, a part or all of computer 100 can be provided on a personal computer and a mobile terminal (a tablet computer, a mobile phone, a smart phone, etc.) In addition, a part or all of user interface 200 can be provided on a personal computer, a mobile terminal, a television, a smart television, etc.

### [Optical Unit 10]

The optical unit 10 includes an optical system for performing OCT measurement and a mechanism for driving predetermined optical elements. The optical system is configured to split light from a light source into measuring light and reference light, generate interference light of returned light of the measuring light and the reference light, and detect the interference light. The optical system has a similar configuration to a conventional spectral domain OCT apparatus. That is, the optical system is configured to split low-coherence light into reference light and measuring light, make the measuring light traveled via an eye E and the reference light traveled via a reference optical path interfere with each other to generate interference light, and detect the spectral components of this interference light. The detection results (detection signals) are transmitted to the computer 100.

In the case of swept source OCT, a wavelength sweeping light source (swept source) is provided instead of a low-coherence light source, while an optical element for spectrally decomposing interference light is not provided. Generally, regarding the configuration of the optical unit 10, known technologies may be applied according to the type of OCT.

The light source 11 outputs broadband, low-coherence light. The low-coherence light includes, for example, a near-infrared wavelength band (approximately 800 nm to 900 nm), and has a temporal coherence length of around several tens of micrometers. It should be noted that a wavelength band that is not visible to human eyes, such as near-infrared light with a central wavelength of around 1040 to 1060 nm, for example, may be used as the low-coherence light.

The light source 11 is configured to include light output device, such as an SLD (super luminescent diode), LED, SOA (Semiconductor Optical Amplifier) or the like.

The low coherence light output from the light source 11 is made into a parallel light flux by a collimate lens 12 and led to a beam splitter 13. The beam splitter 13 may be configured by a half mirror for reflecting a specific ratio of light and transmitting the remaining thereof. The beam splitter 13 divides this parallel light flux into measuring light and reference light.

The measuring light (also referred to as signal light etc.) is to be projected to eye E. An optical element group forming an optical path (a measuring light path) of the measuring light is referred to as a measurement arm (also referred to as a sample arm etc.). The reference light is light acting as a reference for extracting information included in the returned light of the measuring light as interference signals. The optical element group forming an optical path (reference optical path) of the reference light is referred to as a reference arm.

The end of the reference optical path is the beam splitter 13, while the other end thereof is a reference mirror 14. The reference light made up of components passing through the beam splitter 13 is reflected by the reference mirror 14 and returns to the beam splitter 13.

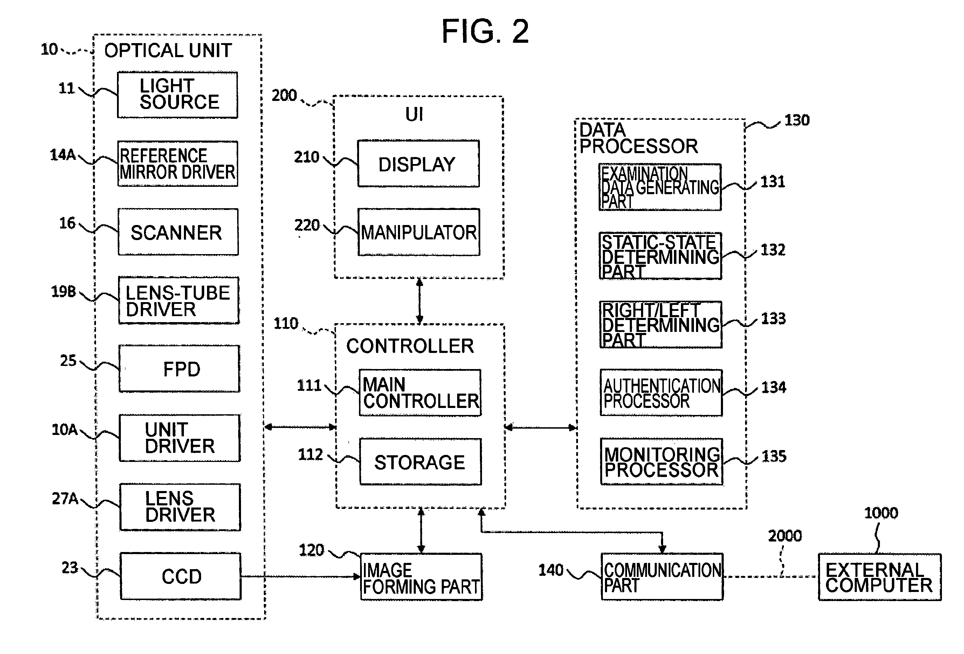

The reference mirror 14 is moved in the travelling direction of the reference light by a reference mirror driver 14A illustrated in Fig. 2. Thereby, the length of the reference optical path is changed. The reference mirror drive 14A functions to relatively change the length of the measuring light path and the length of the reference optical path, changing the depth at which the interference intensity between the measuring light and the reference light becomes highest. The reference mirror 14 and the reference mirror driver 14A serve an example of a maximum interference depth changing part. In addition, the reference mirror driver 14A is an example of a third driver.

In the present embodiment, the configuration to change the length of the reference optical path is applied; however, in place of or in addition to this configuration, the configuration to change the length of the measuring light path can be applied. The change of the length of the measuring light path may be realized with a corner cube for reflecting incident measuring light in the opposite direction of the incident direction and a mechanism for moving this corner cube in the incident direction and the reflection direction.

The measuring light made up of the components reflected by the beam splitter 13 is deflected by a fixed mirror 15 that is inclinedly arranged in the measuring light path, and led to a scanner 16. The scanner 16 is, for example, a biaxial optical scanner. That is, the scanner 16 has the configuration capable of two-dimensionally deflecting measuring light. The scanner 16 is, for example, a mirror scanner including two mirrors that can be mutually perpendicularly deflected. This mirror scanner may be configured with MEMS (Micro Electro Mechanical Systems). As another example, it is possible to configure the scanner 16 using one mirror scanner and one rotary prism.

The measuring light to be output from the scanner 16 is collimated light that has been two-dimensionally deflected. This measuring light is made into focused light by a relay lens 17, and imaged in air on a plane conjugate to a fundus Ef (fundus-conjugate plane) Pc. Further, the measuring light is made into focused light again by an objective lens 19 which functions as a focusing lens, and is incident into the eye E. it should be noted that an optical element arranged in the fundus-conjugate plane Pc (that is, a dichroic mirror 18) is to be described later. In addition, for the case in which a diopter correction lens 27 to be described later is arranged in the measuring light path, the measuring light is refracted by the diopter correction lens 27 after passing through the objective lens 19, and then incident into the eye E.

The objective lens 19 and a lens-tube part 19A are moved by a lens-tube driver 19B illustrated in Fig. 2 along the measuring light path. The objective lens 19 and the lens-tube part 19A are moved in the optical-axis direction in accordance with the refractive power of eye E. Thereby, the fundus-conjugate plane Pc is arranged in the position conjugate to the fundus Ef. As a result, the measuring light is projected to the fundus Ef as a spot light. The objective lens 19 and the lens-tube driver 19B are an example of a focus position changing part for changing the focus position of the measuring light with respect to the eye E. In addition, the lens-tube driver 19B is an example of a first driver that moves the objective lens 19 (focusing lens.) Further, the configuration of providing a focusing lens other than the objective lens 19 can be also applied.

The diopter correction lens 27 is used for changing the focus position of the measuring light with respect to the eye E along with a focusing lens, for example, is an optical element arranged in the measuring light path in order to deal with eyes having excessive refractive power such as excessive myopia eyes. The diopter correction lens 27 is inserted or removed into or from the measuring light path by a lens driver 27A illustrated in Fig. 2. The diopter correction lens 27 and the lens driver 27A are an example of a focus position changing part for changing the focus position of the measuring light with respect to the eye E. In addition, the lens driver 27A is an example of a second driver that moves the diopter correction lens 27.

Further, the focus position changing part may be configured to include multiple diopter correction lenses with different powers and a second driver that selectively arranges any of these diopter correction lenses in the measuring light path. In addition, an optical element such as an Alvarez lens having a variable refractive power can be used as a diopter correction lens. Such an optical element for diopter correction is arranged, for example, between the objective lens 19 and the eye E.

The measuring light projected onto the fundus Ef is scattered (including reflection) at various depth positions of the fundus Ef. The backscattered light (returned light) of the measuring light by the fundus Ef is led to the beam splitter 13 while reversely traveling along the same path as the outward way.

The beam splitter 13 causes the returned light of the measuring light to interfere with the reference light having passed through the reference optical path. At this time, only components of the returned light having passed through a distance that is substantially identical with the length of the reference optical path (in other words, only the backscattered light from the range within a coherent length for the length of the reference optical path) substantially interferes with the reference light. Interference light generated via the beam splitter 13 is led to a spectrometer 20. The interference light entered the spectrometer 20 is dispersed (spectrally decomposed) by a diffracting grating 21, and projected onto a light-receiving surface of a CCD image sensor 23 via a lens 22. It should be noted that the diffracting grating 21 illustrated in Fig. 1 is a transmission type; however, for example, other types of dispersion elements such as a diffracting grating of a reflection type are also available.

The CCD image sensor 23 is for example a line sensor or an area sensor, and detects the respective spectral components of the spectrally decomposed interference light and converts the components into electric charges. The CCD image sensor 23 accumulates these electric charges to generate a detection signal, and transmits this signal to the computer 100.

As described above, the dichroic mirror 18 is inclinedly arranged in the position corresponding to the fundus-conjugate plane Pc in the measuring light path. The dichroic mirror 18 is configured to allow the measuring light in a near-infrared band transmitted therethrough, reflecting light in a visible band.

A flat panel display (FPD) 25 and a lens 26 are provided in the optical path branched from the measuring light path via the dichroic mirror 18. The flat panel display 25 displays information by being controlled by a controller 110. The information to be displayed on the flat panel display 25 may include various visual targets to be presented to the eye E. Examples of such visual targets may include targets for subjective eye examination (Landolt ring etc.) and fixation targets for fixating the eye E.

The flat panel display 25 is arranged in the position conjugate to the fundus-conjugate plane Pc (that is, the position conjugate to the fundus Ef) via the lens 26. As the flat panel display 25, for example, a liquid crystal display (LCD) or organic EL display (OELD) may be used.

Visible light output from the flat panel display 25 is reflected by the dichroic mirror 18 via the lens 26. Further, this visible light enters the eye E via the objective lens 19 and reaches the fundus Ef. Thereby, an image based on this visible light (for example, a visual target image) is projected onto the fundus Ef.

Further, in place of the dichroic mirror 18, an optical element such as a half mirror may be provided. In addition, it is also possible to provide a reflection mirror capable of being inserted or removed into or from the measuring light path. For the case in which the dichroic mirror 18 or the half mirror is provided, it is possible to simultaneously carry out OCT measurement and projection of a visual target. On the other hand, for the case in which the reflection mirror is provided, OCT measurement and projection of a visual target are carried out at different timings.

Although a Michelson-type interferometer is used in this embodiment, it is possible to employ any type of interferometer such as a Mach-Zehnder-type as necessary. Instead of a CCD image sensor, other types of image sensors, such as a CMOS (Complementary Metal Oxide Semiconductor) image sensor, can be used.

According to the present embodiment, light reflected by the beam splitter 13 is used as measuring light, while light transmitted through the beam splitter 13 is used as reference light. In contrast, the configuration can also be applied in which light reflected by the beam splitter 13 is used as reference light and light transmitted through the beam splitter 13 is used as measuring light. In this case, the measurement arm and the reference arm are arranged inversely with Fig. 1.

Members for changing the properties of measuring light and/or reference light can be provided. For example, it is possible to provide an optical attenuator and/or a polarization controller in the reference optical path. The attenuator changes the light intensity of the reference light being controlled by the computer 100. The attenuator includes, for example, a neutral density filter and a mechanism for inserting or removing this neutral density filter into or from the reference optical path. The polarization controller changes the polarization state of the reference light by being controlled by the computer 100. The polarization controller includes, for example, a polarizing plate arranged in the reference optical path and a mechanism for rotating this polarizing plate. Such members are used for adjusting the interference intensity between the returned light of the measuring light and the reference light.

It is possible to provide a front-image-acquiring optical system for imaging the eye E to acquire front images thereof. These front images are the images of an anterior segment or the fundus Ef. The front-image-acquiring optical system forms an optical path branched from the measuring light path, and includes, for example, an illumination optical system and an imaging optical system similar to those of conventional retinal cameras. The illumination optical system irradiates illumination light composed of (near) infrared light or visible light onto the eye E. The imaging optical system detects returned light (reflection light) of the illumination light from the eye E. The imaging optical system has a focusing lens common to the measuring light path (the objective lens 19, the diopter correction lens 27, etc.) and/or a focusing lens independent from the measuring light path. Another example of the front-image-acquiring optical system is an optical system similar to that of conventional SLOs.

For the case in which the front-image-acquiring optical system is installed, it is possible to provide an alignment optical system similar to that of conventional retinal cameras. The alignment optical system forms an optical path branched form the measuring light path and generates a visual target (alignment target) for carrying out position adjustment (alignment) of an apparatus optical system with respect to the eye E. The alignment is position adjustment in the direction (referred to as the xy-direction) perpendicular to the measuring light path (the optical axis of the objective lens 19). The alignment optical system generates two alignment light fluxes from a light flux output from an alignment light source (LED, etc.) by using a two-hole diaphragm (illustration omitted). The two alignment light fluxes are led to the measuring light path via a beam splitter that is inclinedly arranged in the measuring light path, and projected to the cornea of the eye E. Cornea reflection light of the alignment light fluxes is detected by an image sensor of the front-image-acquiring optical system.

For the case in which the alignment optical system is provided, it is possible to carry out automatic alignment. Specifically, a data processor 130 of the computer 100 analyzes signals input from the image sensor of the front-image-acquiring optical system to identify the positions of two alignment target images. Further, based on the identified positions of the two alignment target images, the controller 110 moves the optical unit 10 in the xy-direction such that two cornea reflection lights are projected while overlapping each other at a specific position (for example, the center position) of the light-receiving surface of the image sensor. Here, the optical unit 10 is moved by a unit driver 10A.

In addition, for the case in which the front-image-acquiring optical system is installed, it is possible to provide a focus optical system similar to that of conventional retinal cameras. The focus optical system forms an optical path branched from the measuring light path, and generates a visual target (split target) for carrying out focusing on the fundus Ef (focus adjustment). The focus optical system generates two focusing light fluxes from a light flux output from a focusing light source (LED, etc.) by using a split target plate (illustration omitted). The two focusing light fluxes are led to the measuring light path via a reflection member that is inclinedly arranged on the measuring light path, and projected onto the fundus Ef. The fundus reflection light of the focusing light fluxes is detected by the image sensor of the front-image-acquiring optical system.

For the case in which the focus optical system is provided, automatic focusing can be carried out. Specifically, the data processor 130 of the computer 100 analyzes signals input from the image sensor of the front-image-acquiring optical system to identify the positions of two split-target images. Further, based on the identified positions of the two alignment target images, the controller 110 controls movements of the focus optical system and the focusing lens (for example, movement of the objective lens 19) such that two fundus reflection lights are projected in alignment onto the light-receiving surface of the image sensor.

For the case in which the front-image-acquiring optical system is provided, it is possible to carry out automatic tracking. Automatic tracking is a function to move the optical unit 10 in accordance with the movement of the eye E. In the case of carrying out automatic tracking, alignment and focusing are carried out in advance. For example, automatic tracking is carried out as follows. First, the front-image-acquiring optical system starts taking a moving picture of the eye E. The data processor 130 monitors the movement of the eye E (changes in the position) by sequentially analyzing frames to be acquired by the moving picture shooting. The controller 110 controls the unit driver 10A to move the optical unit 10 in accordance with the position of the eye E to be sequentially acquired. Thereby, it is possible to allow the optical unit 10 to follow the movement of the eye E in real time, making it possible to maintain a preferable positional relation in which alignment and focus have been adjusted.

### [Control system and data processing system]

The control system and the data processing system of the ophthalmologic imaging apparatus 1 according to the embodiment will be described. An example of the configuration of the control system and the data processing system is illustrated in Fig. 2.

The computer 100 is the center of the control system and the data processing system. The computer 100 comprises a microprocessor, RAM, ROM, a hard disk drive, a communication interface, etc. A storage device such as a hard disk drive etc. stores computer programs for allowing the ophthalmologic imaging apparatus 1 to carry out various processing. The computer 100 may have a dedicated circuit board for executing specific processing. For example, a circuit board for executing the processing to form OCT images may be provided.

### (User interface 200)

The user interface 200 is connected to the computer 100. The user interface 200 includes a display 210 and a manipulator 220. The display 210 is configured including a display device such as a FPD. The manipulator 220 is configured including operation devices such as a button, a key a joy stick and an operation panel provided on the case of the ophthalmologic imaging apparatus 1 or outside thereof. For the case in which the computer 100 includes a personal computer, the manipulator 220 may include operation devices of this personal computer (a mouse, a keyboard, a track pad, a button, etc.)

The display 210 and the manipulator 220 do not need to be configured as separate devices. For example, like a touch panel, a device can be used in which a display function and an operation function are integrated. In such cases, the manipulator 220 may be configured to include the touch panel and computer programs. The content of operation via the manipulator 220 is input to the controller 110 as an electric signal. Moreover, operations and inputs of information may be performed by using a graphical user interface (GUI) displayed on the display 210 and the manipulator 220.

### (Controller 110)

The controller 110 is provided in the computer 100. The controller 110 comprises a microprocessor, RAM, ROM, a hard disk drive, etc. A main controller 111 and storage 112 are provided in the controller 110.

### (Main controller 111)

The main controller 111 controls respective parts of the ophthalmologic imaging apparatus 1. For example, the control targets of the main controller 111 includes the unit driver 10A, the light source11 the reference mirror driver 14A, the scanner 16, the lens-tube driver 19B, the CCD (image sensor) 23, the flat panel display 25, the display 210, the data processor 130, and a communication part140.

The unit driver 10A has a mechanism for moving the optical unit 10 in the direction (z-direction) along the measuring light path (the optical axis of objective lens 19) and in the direction (xy-direction) along a surface orthogonal to the z-direction. The reference mirror driver 14A moves the reference mirror 14 along the reference optical path. The lens-tube driver 19B moves the objective lens 19 and the lens-tube part 19A along the measuring light path. The lens driver 27A inserts or removes the diopter correction lens 27 into or from the measuring light path. Alternatively, the lens driver 27A selectively arranges multiple diopter correction lenses in the measuring light path (it can also remove all diopter correction lenses from the measuring light path).

### (Storage 112)

The storage 112 stores various data. In addition, the storage 112 stores various programs and data for operating the ophthalmologic imaging apparatus 1. The data to be stored in storage 112 includes data to be acquired by the ophthalmologic imaging apparatus 1 and data stored in advance.

The data to be acquired by ophthalmologic imaging apparatus 1 may include image data of OCT images, examination data, and image data of front images, etc. the examination data may be a data representing a state of the eye that is generated by processing detection results of interference light from the optical unit 10 (details to be described later). The data stored in advance in the storage 112 may include setting information, authorized personal authentication information, etc.

### (Setting information)

The setting information comprises information in which contents of settings of specific items regarding the optical unit 10 and the data processor 130 are recorded. The setting information includes, for example, a setting content(s) regarding at least one of the following items: (1) fixation position; (2) scanning pattern; (3) focus position; (4) diopter correction; (5) maximum interference depth; and (6) analysis processing.
(1) "Fixation position" means a direction in which the eye E is fixated, that is, the site of the eye E in which OCT measurement is to be conducted. The choices of the fixation position may include the fixation position for conducting OCT measurement of a macula and its surroundings, the fixation position for conducting OCT measurement of an optic disc and its surroundings, and the fixation position for conducting OCT measurement of a macula and an optic disc and their surroundings, etc. In addition, it is also possible to set the fixation position corresponding to arbitrary site of the eye E. The fixation position may be specified, for example, from information indicating a displayed position (a position of a pixel) of the fixation target on the flat panel display 25.
(2) "Scanning pattern" indicates in what pattern the projection position of the measuring light to the eye E is moved. The choices of the scanning pattern may include one or more line scans (horizontal scan, vertical scan), one or more cross scans, radial scan, circle scan, etc. In addition, for the case of acquiring three-dimensional images (three-dimensional data set), a three-dimensional scan in which intervals between multiple line scans are sufficiently narrow.
(3) "Focus position" indicates a focus condition that is applied in OCT measurement. The focus position may be specified, for example, by information indicating the position of the objective lens 19.
(4) "Diopter correction" indicates a condition that is applied in the diopter correction. Specifically, the condition may include a value showing the refractive power (visual acuity value) of the eye E, application or non-application of the diopter correction lens, and/or a value showing the refractive power applied by the diopter correction lens, etc.
(5) "Maximum interference depth" indicates the depth at which the interference intensity between the measuring light and the reference light becomes highest in OCT measurement. The maximum interference depth includes, for example, information indicating the position of the reference mirror 14.
(6) "Analysis processing" means contents of processing to be carried out based on data to be acquired by the optical unit 10, that is, types of examination data to be acquired. Fundus-layer-thickness analysis, drusen analysis, disc-shape analysis, etc. are exemplary of the analysis processing. The fundus-layer-thickness analysis is the analysis processing for acquiring the thickness of a specific fundus layer tissue (retina, sub tissue of retina, choroid, sclera, etc.). Drusen analysis is the analysis processing for acquiring the distribution of drusen (mass of waste) to be used as diagnostic materials for age-related macular degeneration. The disc-shape analysis is the analysis processing for acquiring the shape of optic disc by detecting a hole of retina (cuts, defective sites, etc.) through the analysis of a cross-sectional image and/or a three-dimensional image. In addition, the inclination of the optic disc (asymmetric property of the shape) can be also acquired through the disc-shape analysis. The details of these analysis processing are to be described later.

For the case in which OCT measurement of both a left eye and a right eye of a subject is conducted, particularly, for the case in which different settings are applied to the right eye and the left eye, it is possible to separately provide the setting information for the left eye and the setting information for the right eye.

In addition, for the case in which the ophthalmologic imaging apparatus 1 is shared by two or more subjects, particularly, for the case in which different settings are applied depending on the subject, it is possible to provide individual setting information for each subject.

An example of a method of creating the setting information will be described. The ophthalmologic imaging apparatus 1 is lent to a subject(s) and used at the subject's residence. The setting information is created before the lending.

As a first example of the creation method of the setting information, the user interface 200 of the ophthalmologic imaging apparatus 1 can be used. For example, the setting contents of specific items regarding the optical unit 10 and the data processor 130 are input to a specific setting screen displayed on the display 210 by using the manipulator 220. The main controller 111 creates setting information including the input setting contents, and stores it in the storage 112. In this case, the user interface 200 functions as an interface part.

A computer (external computer 1000) capable of connecting to the ophthalmologic imaging apparatus 1 can be used as a second example of the creation method of the setting information. The external computer 1000 is, for example, a personal computer used by a doctor. External computer 1000 is equipped with a function (computer program) for creating setting information. A specific setting screen is displayed on a display of the external computer 1000. A doctor etc. inputs setting contents of specific items regarding the optical unit 10 and the data processor 130 to this setting screen by using operation devices (keyboard, mouse, etc.). The external computer 1000 transmits the input setting contents to the ophthalmologic imaging apparatus 1 via a communication line 2000 (or a cable for direct connection). The ophthalmologic imaging apparatus 1 receives the setting contents transmitted from the external computer 1000 via the communication part 140. The main controller 111 creates setting information including the received setting contents, and stores it in the storage 112. In this case, the communication part 140 functions as an interface part. It should be noted that, in the above-described example, the external computer 1000 inputs and transmits the setting contents; however, the external computer 1000 may be configured to create setting information in addition to inputting and transmitting setting contents.

Upon creating the setting information, examination results, examination conditions, disease names (such as the type of data that are diagnostic materials), etc. of the eye E are referred. For example, the fixation position is set with reference to the fixation position applied to a past OCT measurement and/or a disease name. The scanning pattern is set with reference to the scanning pattern applied to a past OCT measurement and a disease name. The focus position is set with reference to the focus position applied to a past OCT measurement. The diopter correction is set with reference to whether or not the diopter correction lens is applied to a past OCT measurement, or with reference to the visual acuity value and/or the refractive power of the eye E acquired via a past examination. The maximum interference depth is set with reference to the maximum interference depth applied to a past OCT measurement. The analysis processing is set with reference to the type of analysis processing applied to a past examination and a disease name.

A specific example of relations between the examination results, examination conditions and/or disease names, and the setting content will be described. The examination of a macula can adopt the following setting content: (1) as the fixation position, the fixation position such that the macula is included in a scanning range, for example, the fixation position such that the macula is located on an extension line of the optical axis of the measuring light path, is adopted; (2) as the scanning pattern, three-dimensional scan, radial scan and/or line scan are/is adopted; (3) as the focus position, the focus position applied in a past OCT measurement or the focus position acquired from the measurement value of the eye E (axial length, a refractive power, etc.) through calculation is adopted; (4) as the diopter correction, the presence or absence of application of the diopter correction lens in a past OCT measurement or the diopter correction value acquired from the measurement value of a refractive power of the eye E, etc. is adopted; (5) as the maximum interference depth, the maximum interference depth applied in a past OCT measurement or the maximum interference depth acquired from the measurement value of the eye E (axial length, a refractive power, etc.) through calculation is adopted. In this case, it is possible to refer to the site of the fundus Ef that is set as the maximum interference depth (such as the fundus surface, the deep tissue to be watched); and (6) as the analysis processing, fundus-layer-thickness analysis (and comparison analysis with a standard layer thickness value) is employed. Further, according to this fundus-layer-thickness analysis, for example, the thickness of the retina is derived (retinal thickness analysis).

The examination of an optic disc can adopt the following setting content: (1) as the fixation position, the fixation position such that the optic disc is included in a scanning range, for example, the fixation position such that the optic disc is located on an extension line of the optical axis of the measuring light path, is adopted; (2) as the scanning pattern, three-dimensional scan and/or circle scan are/is adopted; (3) as the focus position, the focus position applied in a past OCT measurement or the focus position acquired from the measurement value of the eye E (axial length, a refractive power, etc.) through calculation is adopted; (4) as the diopter correction, the presence or absence of application of the diopter correction lens in a past OCT measurement or the diopter correction value acquired from the measurement value of a refractive power of the eye E, etc. is adopted; (5) as the maximum interference depth, the maximum interference depth applied in a past OCT measurement or the maximum interference depth acquired from the measurement value of the eye E (axial length, a refractive power, etc.) through calculation is adopted. In this case, it is possible to refer to the site of the fundus Ef that is set as the maximum interference depth (such as the fundus surface, the deep tissue to be watched (for example, the retinal nerve fiber layer)); and (6) as the analysis processing, fundus-layer-thickness analysis (and comparison analysis with a standard layer thickness value) and/or disc-shape analysis are/is employed. Further, according to this fundus-layer-thickness analysis, for example, the thickness of the retinal nerve fiber layer is derived (RNFL analysis).

The examination of glaucoma can adopt the following setting content: (1) as the fixation position, the fixation position such that a macula is included in a scanning range (for example, the fixation position such that the macula is located on an extension line of the optical axis of the measuring light path) and/or the fixation position such that an optic disc is included in a scanning range (for example, the fixation position such that the optic disc is located on an extension line of the optical axis of the measuring light path) are/is adopted; (2) as the scanning pattern, three-dimensional scan is adopted; (3) as the focus position, the focus position applied in a past OCT measurement or the focus position acquired from the measurement value of the eye E (axial length, a refractive power, etc.) through calculation is adopted; (4) as the diopter correction, the presence or absence of application of the diopter correction lens in a past OCT measurement or the diopter correction value acquired from the measurement value of a refractive power of the eye E, etc. is adopted; (5) as the maximum interference depth, the maximum interference depth applied in a past OCT measurement or the maximum interference depth acquired from the measurement value of the eye E (axial length, a refractive power, etc.) through calculation is adopted. In this case, it is possible to refer to the site of the fundus Ef that is set as the maximum interference depth (such as the fundus surface, the deep tissue to be watched (for example, the retinal nerve fiber layer)); and (6) as the analysis processing, retinal thickness analysis (and comparison analysis with a standard layer thickness value), RNFL analysis (and comparison analysis with a standard layer thickness value) and/or disc-shape analysis are/is employed.

The examination of age-related macular degeneration can adopt the following setting content: (1) as the fixation position, the fixation position such that a macula is included in a scanning range, for example, the fixation position such that the macula is located on an extension line of the optical axis of the measuring light path is adopted; (2) as the scanning pattern, three-dimensional scan is adopted; (3) as the focus position, the focus position applied in a past OCT measurement or the focus position acquired from the measurement value of the eye E (axial length, a refractive power, etc.) through calculation is adopted; (4) as the diopter correction, the presence or absence of application of the diopter correction lens in a past OCT measurement or the diopter correction value acquired from the measurement value of a refractive power of the eye E, etc. is adopted; (5) as the maximum interference depth, the maximum interference depth applied in a past OCT measurement or the maximum interference depth acquired from the measurement value of the eye E (axial length, a refractive power, etc.) through calculation is adopted. In this case, it is possible to refer to the site of the fundus Ef that is set as the maximum interference depth (such as the fundus surface, the deep tissue to be watched); and (6) as the analysis processing, retinal thickness analysis (and comparison analysis with a standard layer thickness value) and/or drusen analysis are/is employed.

It is also possible to automatically create a part or all of the setting information. In this case, the main controller 111 or the external computer 1000 acquires the examination results, the examination conditions and/or the information regarding the disease names of the eye E from an electronic medical chart of the present subject. Subsequently, the setting information including the acquired information is created.

### (Authorized personal authentication information)

The authorized personal authentication information is the personal authentication information for a person who is allowed to conduct the examination using the ophthalmologic imaging apparatus 1 (authorized subject). The personal authentication information is used for carrying out personal authentication of a person intending to conduct the examination using the ophthalmologic imaging apparatus 1.

Character string information and/or image information may be used as the personal authentication information. A patient ID provided from a medical institute, the individual information such as the name of a subject, the character string information arbitrarily designated by a subject, the randomly-designated character string information, etc. are exemplary of the character string information. Biometric authentication information (a fingerprint pattern, an iris pattern, a venous pattern, a facial form pattern, etc.), one-dimensional code, two-dimensional code, etc. are exemplary of the image information. In addition, a voice pattern and a handwriting pattern can be also used as the personal authentication information.

The authorized personal authentication information is input into the ophthalmologic imaging apparatus 1 before the ophthalmologic imaging apparatus 1 is lent to a subject. The input method when the authorized personal authentication information is the character string information includes manual entry using the user interface 200 or the external computer 1000, read entry using a reading device (reader) such as a card reader, read-in entry from an electronic medical chart etc. In addition, the input method when the authorized personal authentication information is the image information includes read entry using a reading device (reader) such as a card reader, scan entry of the information described on a leaf, read-in entry from an electronic medical chart etc., entry from a biometric authentication information input device (a fingerprint reader, an iris reader, a venous reader, a facial form analyzer, etc.). In addition, authorized personal authentication information is input from a voice-input device when a voice pattern is used. In addition, authorized personal authentication information is input from a scanner that reads the information described on a leaf when a handwriting pattern is used.

A person intending to conduct the examination using the ophthalmologic imaging apparatus 1 inputs the personal authentication information according to a specific method. The input method corresponds to the type of personal authentication information to be used. In other words, entry of the personal authentication information to be used when using the ophthalmologic imaging apparatus 1 is carried out according to the same method as the above-described entry of the authorized personal authentication information. The composition element for inputting personal authentication information corresponds to a second input part.

Specific examples of the second input part include the followings: the user interface 200 for manual entry of the personal authentication information; the communication part 140 for receiving the personal authentication information input in the external computer 1000; a reader such as a card reader for reading the personal authentication information recorded in a recording medium such as a card; the communication part 140 for receiving the personal authentication information recorded in an electronic medical chart etc.; a scanner for scanning the personal authentication information described on a leaf; a biometric authentication information input device (a fingerprint reader, an iris reader, a venous reader, a facial form analyzer, etc.) for reading the personal authentication information made of biometric authentication information; and a voice-input device for inputting the personal authentication information made up of audio information.

For the case in which the ophthalmologic imaging apparatus 1 is shared by two or more subjects, the authorized personal authentication information of each of the subjects is stored in the storage 112 in advance.

### (Image forming part 120)

An image forming part 120 forms image data of a cross sectional image of the eye E based on the detection signals from the CCD image sensor 115. Like the conventional Fourier-Domain OCT, this process includes processes such as noise elimination (noise reduction), filtering, dispersion compensation and FFT (Fast Fourier Transform). In the case of other types of OCT apparatus, the image forming part 120 executes known processes in accordance with the type thereof.

The image forming part 120 is configured to include, for example, a dedicated circuit board and/or a microprocessor. It should be noted that "image data" and the "image" based on this may be identified with each other in the specifications.

### (Data processor 130)

A data processor 130 executes various data processes. For example, the data processor 130 executes image processes on images formed by the image forming part 120. As an example thereof, the data processor 130 is capable of forming image data of a three-dimensional image of the eye E based on multiple two-dimensional cross sectional images at different cross sections. The image data of a three-dimensional image means an image data in which the position of a pixel is defined by a three-dimensional coordinate system. The image data of a three-dimensional image may be an image data having voxels that are three-dimensionally arranged. This image data is referred to as volume data, voxel data, or the like. For displaying an image based on volume data, the data processor 130 executes a rendering process (such as volume rendering and MIP (Maximum Intensity Projection)) on this volume data to form image data of a pseudo three-dimensional image taken from a specific view direction. Further, the data processor 130 is capable of imaging an arbitrary cross section of a three-dimensional image (MPR (Multi-Planar Reconstruction)).

Further, it is also possible to form stack data of multiple cross sectional images as image data of a three-dimensional image. Stack data is image data obtained by three-dimensionally arranging multiple cross sectional images obtained along multiple scanning lines, based on the positional relation of the scanning lines. That is to say, stack data is image data obtained by expressing multiple cross sectional images defined by originally individual two-dimensional coordinate systems by a three-dimensional coordinate system (namely, embedding into a three-dimensional space). The data processor 130 is capable of carrying out MPR based on stack data.

The data processor 130 includes, for example, a microprocessor, RAM, ROM, hard disk drive, dedicated circuit board for a specific processing, and so on. Computer programs that cause a microprocessor to execute the data processing stated below are previously stored in a storage device such as a hard disk drive.

The data processor 130 comprises an examination data generating part 131, a static-state determining part 132, a right/left determining part 133, an authentication processor 134, and a monitoring processor 135.

### (Examination data generating part 131)

The examination data generating part 131 generates examination data indicating the state of the eye E by processing the detection results of the interference light from the optical unit 10. The examination data generating part 131 is an example of a processor. "The detection results of the interference light" processed by the examination data generating part 131 are, for example, any of the followings: (1) signals output from the CCD image sensor 23; (2) image data formed by the image forming part 120; (3) data acquired at the middle stage of the processing carried out by the image forming part 120 (that is, the data acquired in the middle of image data forming processing); (4) data acquired by processing signals output from the CCD image sensor 23 via a composition element other than the image forming part 120.

An example of the processing carried out by the examination data generating part 131 will be described. As a first example, the examination data generating part 131 can generate layer thickness information of the fundus Ef based on the detection results of the interference light from the optical unit 10. In this case, the examination data generating part 131 functions as a layer thickness information generating part, carrying out the above-mentioned fundus-layer-thickness analysis (retinal thickness analysis, RNFL thickness analysis, etc.) Further, the examination data generating part 131 can carry out comparative analysis between the layer thickness information acquired by the fundus-layer-thickness analysis and the standard layer thickness value.

The fundus-layer-thickness analysis is a processing for deriving (distribution of) the thickness of a specific layer tissue of the fundus Ef based on the detection results of the interference light. The following is an explanation of the retinal thickness analysis as an example thereof. For the case in which the thickness of other layer tissues is derived, a similar processing is carried out.

In the retinal thickness analysis, the thickness distribution of the retina in a part or all of a scanning area is obtained by analyzing a cross sectional image or a three-dimensional image of the fundus Ef, for example. It should be noted that there are various definitions of retinal thickness. For example, the thickness from the inner limiting membrane to the inner granular layer (photoreceptor inner and outer segments: IS/OS) may be defined as the retinal thickness, or the thickness from the inner limiting membrane to the pigment epithelial layer of the retina may be defined as the retinal thickness. The retinal thickness obtained by the retinal thickness analysis can be of any of these definitions.

The retinal thickness analysis is executed in the following manner, for example. First, an OCT image of the fundus Ef is analyzed, and an image region corresponding to prescribed boundary regions (e.g., the inner limiting membrane and the pigment epithelial layer of the retina) is identified. Then, the number of pixels between the identified boundary regions is counted to obtain the retinal thickness (i.e. distance in the direction of depth). Note that the process of analyzing an OCT image and obtaining the thickness of the fundus layer is also described in Japanese published unexamined application 2007-325831, Japanese published unexamined application 2008-206684, Japanese published unexamined application 2009-61203, and Japanese published unexamined application 2009-66015, etc. by the present applicants.

The comparison analysis of retinal thickness is an analytic process that compares the retinal thickness obtained through the retinal thickness analysis with standard data (normative data) stored in advance. The normative data are standard values of the retinal thicknesses (standard thickness) of healthy eyes. The normative data are prepared by measuring the retinal thicknesses of multiple healthy eyes, and obtaining statistical values (mean value, standard deviation etc.) of the measurement results. The comparison analysis determines whether or not the retinal thickness of the subject eye E is within the range of the retinal thicknesses of healthy eyes. The comparison analysis may be performed by obtaining the range of the retinal thicknesses of eyes with a disorder and determining whether or not the retinal thickness obtained through the retinal thickness analysis is within this range.

The examination data generating part 131 may be configured to be capable of carrying out the drusen analysis. The drusen analysis is an analytic processing for obtaining the state of distribution of drusen in a scanning area by analyzing an OCT image. This state of distribution includes the position and/or size (area, volume, diameter, etc.) of the drusen in the fundus Ef.

The drusen analysis may be executed by, for example, analyzing an OCT image and identifying an image region corresponding to Bruch's membrane and an image region corresponding to the retinal pigment epithelial layer, and based on the pixel values between these image regions, identifying image regions corresponding to small, roughly circular raised configurations as (candidates of) drusen. This type of identification process of image regions based on shape may be performed through image matching with a template of the relevant shape, for example. Based on the identified image region corresponding to drusen, the examination data generating part 131 can derive the position, number, size, etc. of the drusen. Further, based on the derived distribution of the drusen, the examination data generating part 131 can generate evaluation information of the state of age-related macular degeneration.

In the case in which the aforementioned front-image-acquiring optical system is provided and photography of the fundus Ef is allowed, it is possible to carry out the drusen analysis based on a captured image of the fundus Ef. This drusen analysis is executed by, for example, determining whether the pixel values of each pixel of the captured image are within a prescribed range, and identifying pixels included in the prescribed range. If the captured image is a color image, because drusen is depicted with a characteristic color (pale yellow), the range of pixel values corresponding to this characteristic color is preliminarily set as the above prescribed range. If the captured image is a monochromatic image, because drusen is depicted with a characteristic brightness, the range of pixel values corresponding to this characteristic brightness is preliminarily set as the above prescribed range. Further, it is possible to specify an image region corresponding to drusen by executing template matching based on the standard shape of drusen (small, roughly circular raised shape).

The disc-shape analysis may include an analytic processing that analyzes a cross sectional image or a three-dimensional image of the fundus Ef to detect openings (cuts, defective regions) in the retina, and obtains the shape of the optic disc. For example, in the disc-shape analysis, an image region corresponding to the retina surface of the optic disc and its vicinity by analyzing a cross sectional image etc. is identify, and parameters (disc-shape parameters) representing its global shape and/or local shape (irregularities) by analyzing the identified image region. Examples of disc-shape parameters include the cup diameter, the disc diameter, the rim diameter, and the depth of the optic disc, etc.

In addition, the disc-shape analysis may include analysis processing for acquiring the tilt of an optic disc (asymmetric property of the disc shape). This analysis processing is carried out, for example, as follows. First, the examination data generating part 131 identifies the center of the disc by analyzing a three-dimensional image obtained through scanning of a region including the optic disc. Subsequently, the examination data generating part 131 sets a circle region centering on the disc center and acquires multiple partial regions by radially dividing this circular region. Subsequently, the examination data generating part 131 acquires the height position of a specific layer (for example, retinal pigment epithelial layer) at each pixel position by analyzing the cross-sectional image of the circular region. Further, the examination data generating part 131 calculates the average value of the height positions of the specific layer in the respective partial regions. Subsequently, the examination data generating part 131 compares a pair of average values acquired for a pair of partial regions corresponding to the opposing positions with respect to the disc center to acquire the inclination of the fundus Ef in this opposing direction. Subsequently, the examination data generating part 131 generates inclination distribution information indicating the distribution of inclinations of the fundus Ef in the above-mentioned circular region based on the inclinations acquired for the multiple opposing directions. Further, it is possible to generate evaluation information of the disease status based on the generated inclination distribution information (and the information indicating its standard distribution).

The above-described examination data is based on the results of OCT measurements; however, examination data may include the results of other examinations. This is exemplified by the case in which the flat panel display 25 can display a visual target (Landolt ring, etc.) for subjective eye examination, whereby the examination data generating part 131 can generate examination data including the results of the subjective eye examination.

Subjective eye examination is conducted through the subject's responses to visual targets that are presented to the eye E. the examination data generating part 131, according to a specific computer program, repeatedly conducts processing for determining right or wrong with regard to the response from the subject as well as processing for selecting a visual target to be subsequently presented in accordance with the determination results. The main controller 111 causes the flat panel display 25 to display the visual target selected by the examination data generating part 131. The examination data generating part 131 derives the visual acuity value of the eye E by repeatedly carrying out such processing, generating examination data including this visual acuity value.

### (Static-state determining part 132)

The static-state determining part 132 determines whether or not the eye E remains substantially still based on the data acquired by the optical unit 10 (static-state determination processing). "Remains substantially still" includes not only the case in which the eye E remains still but also the case in which the eye E moves without much influence on OCT measurement. The permissible range of this movement has been arbitrarily set.

Examples of static-state determination processing will be described. A first example of this is static-state determination processing based on the intensity of the returned light of the measuring light. The intensity of the returned light of the measuring light becomes highest in the status in which the alignment is matched (as the regular reflection from the cornea becomes the highest). The intensity of the returned light can be detected, for example, by detecting a part of the returned light by a photo detector etc. The static-state determining part 132 can determine whether or not the eye E remains substantially still based on temporal changes in the intensity of the returned light. Alternatively, as the intensity of the returned light affects the intensity of the interference light, it is possible to carry out static-state determination based on temporal changes in the intensity of signals from the CCD image sensor 23.

As a second example, for the case in which the above-mentioned front-image-acquiring optical system is provided, it is possible to carry out the following static-state determination processing. First, the front-image-acquiring optical system takes a moving picture of the eye E. Thereby, front images (frames) of the eye E are acquired at specific time intervals. The static-state determining part 132 detects a characteristic site of the eye E by analyzing sequentially-input front images. This characteristic site is, for example, a pupil (or its center) in anterior segment images, and is, for example, the optic disc (or its center), the macula (or its center), a blood vessel, and a diseased site in fundus images. Further, the static-state determining part 132 can determine whether or not the eye E remains substantially still by monitoring changes in the position of the characteristic site in the front images to be input in chorological order.

### (Right/left determining part 133)

The right/left determining part 133 determines whether the eye E is a right eye or a left eye (right and left determination processing). The right and left eye determination processing is carried out in the case of conducting the examination on both of the right and left eyes using the ophthalmologic imaging apparatus 1. When an examination is conducted for only one of the right or left eyes, for example, the information indicating if the target eye of the examination is the left eye or the right eye is stored in the storage 112 in advance.

Further, even if only one eye is the examination target, right/left determination processing may be carried out in order to avoid the situation in which the examination of the other eye is conducted by mistake. For example, for the case in which the left eye is set as the examination target, and if the eye E is determined to be a right eye as a result of the right/left determination processing, the right/left determining part 133 outputs specific annunciation information. This annunciation information is, for example, display information output by the display 210 or the flat panel display 25, or audio information output by the unillustrated voice output part. In addition, when the measuring light includes visible components, it is possible to carry out announcement, for example, by blinking the measuring light.

Examples of the right/left determination processing will be described. A first example of this is right/left determination processing based on the control status for the unit driver 10A. The present example is employed for the case in which the position of the optical unit 10 differs between the examination of the right eye and that of the left eye. As described above, the optical unit 10 is moved by the unit driver 10A under the control of the main controller 111. The main controller 111 transmits the control details to the right/left determining part 133 every time it controls the unit driver 10A. The right/left determining part 133 determines whether the optical unit 10 is arranged in the position for examining the left eye or the position for examining the right eye based on the control details received from the main controller 111. Here, the range of positions for examining the left eye and the range of positions for examining the right eye have been respectively set in advance.

As a second example, it is possible to carry out right/left determination processing by analyzing front images for the case in which the above-mentioned front-image-acquiring optical system is provided. When the front image is an anterior segment image, for example, based on the shape of an eyelid, it is capable of identifying the inner and outer corners of the eye, making it possible to determine whether the eye E is the left eye or the right eye. In addition, when the front image is a fundus image, it is possible to determine if the eye E is the left eye or the right eye depending on the position of the optic disc, the position of the macula, the positional relation between the optic disc and the macula, the running condition of the blood vessels, etc.

As described above, the right/left determining part 133 functions to automatically determine if the eye E is a left eye or a right eye. The determination result is input into the controller 110. The right/left determining part 133 functions as a first input part for inputting information indicating if the eye E is a left eye or a right eye into the controller 110. On the other hand, the configuration without such an automatic determination function is also available. For example, the subject (or his or her assistant) inputs the information indicating if the eye E is a left eye or a right eye via the manipulator 220. In this case, the manipulator 220 corresponds to a first input part.

### (Authentication processor 134)

As described above, the controller 110 receives the personal authentication information from the second input part. The controller 110 transmits the input personal authentication information to the authentication processor 134 together with the authorized personal authentication information stored in the storage 112. The authentication processor 134 determines whether or not this personal authentication information and the authorized personal authentication information coincide with each other. The authentication processor 134 transmits its determination result to the controller 110.

When the ophthalmologic imaging apparatus 1 is shared by two or more subjects, that is, when two or more authorized subjects exist, as described above, authorized personal authentication information for each subject is stored in the storage 112. The controller 110, upon reception of entry of the personal authentication information, transmits all authorized personal authentication information stored in the storage 112 to the authentication processor 134 together with the received personal authentication information. The authentication processor 134 determines whether or not the received personal authentication information coincides with any of the authorized personal authentication information. In other words, the authentication processor 134 explores the authorized personal authentication information coinciding with the received personal authentication information.

### (Monitoring processor 135)

The monitoring processor 135 monitors the operational status of a specific site of the ophthalmologic imaging apparatus 1. For example, the monitoring processor 135 detects a malfunction, a failure, a breakage, etc. of the specific site of the ophthalmologic imaging apparatus 1. Alternatively, the monitoring processor 135 detects that the specific site of ophthalmologic imaging apparatus 1 is likely to have a malfunction, failure, or breakage. As a specific example of this, the monitoring processor 135 measures accumulative operational time, and detects that its measurement result exceeds a specific threshold.

Sites of the ophthalmologic imaging apparatus 1 considered as a monitoring target may include any hardware and/or any software. Such hardware may include a microprocessor, RAM, ROM, a hard disk drive, a communication interface, a light source, optical elements, light receiving elements, an actuator, a mechanism, a cable, etc. Such software may include computer programs for controlling the apparatus, computer programs for data processing, etc.

Examples of a monitoring method of the operational status of the specific site will be described. The monitoring processor 135 detects a physical amount relating to hardware of the monitoring target, determining whether or not the hardware is abnormal by determining whether or not the detected value is within a permissible range. Examples of such processing may include the following: detecting heat, then, determining whether or not this heat is equal to or more than a specific temperature; detecting sound generated by a mechanism, then, determining whether or not this sound is an abnormal sound according to its frequency etc.; detecting displacement of hardware via an encoder etc. then, determining whether or not the abnormal operation or backlash of the mechanism is generated.

As another example of the monitoring method, specific data is input into a microprocessor, making it possible to determine whether or not the microprocessor is operating normally or whether or not computer programs are normal depending on if the processed data is normal or not.

### (Communication part 140)

The communication part 140 performs data communication with external devices. The data communication system is optional. For example, the communication part 140 may include a communication interface based on the Internet, a communication interface based on LAN, a communication interface based on short-range communication, etc. In addition, data communication may be wired communication or wireless communication.

The communication part 140 performs data communication with the external computer 1000 defined in advance via the communication line 2000. Further, an arbitrary number, at least one, of the external computers 1000 can be provided. As the external computer 1000, servers placed in the medical institute, terminals used by doctors, servers of a manufacturer (or a sales company, a maintenance company, a rental company, etc.) of the ophthalmologic imaging apparatuses 1, terminals used by a person in the manufacture, etc. are cited.

The data to be transmitted or received by the communication part 140 may be encoded. In this case, the controller 110 (or the data processor 130) has an encoding processer for encoding the transmission data and a decoding processor for decoding the reception data.

### [Operations]

Operations of the ophthalmologic imaging apparatuses 1 according to the embodiment will be explained.

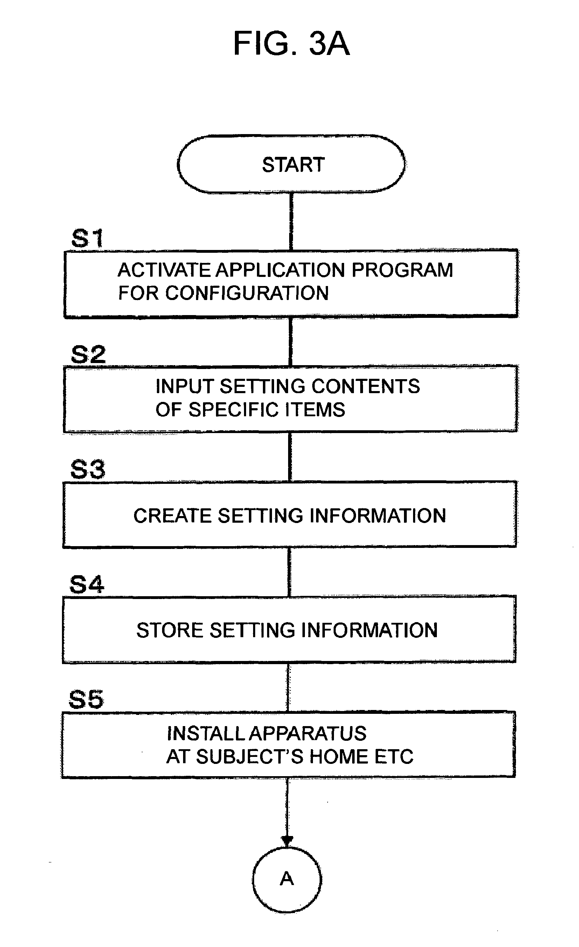

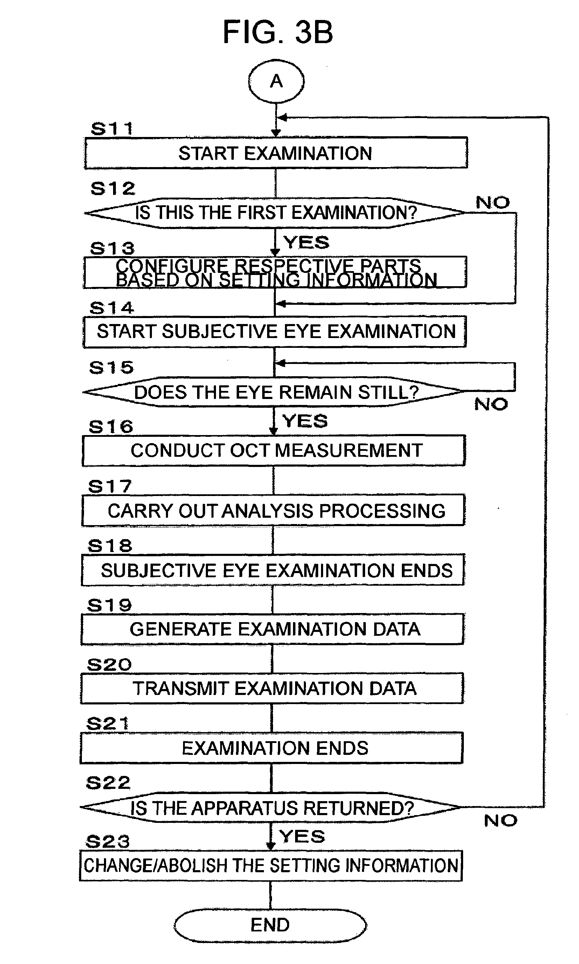

An operational example of the ophthalmologic imaging apparatus 1 is illustrated in Fig. 3A and Fig. 3B. In this operational example, the ophthalmologic imaging apparatus 1 is installed at a subject's home to be used after it is configured at a medical institute etc. Manipulation of the ophthalmologic imaging apparatus 1 is carried out by the subject (or his or her assistant). Fig. 3A illustrates a flow until the ophthalmologic imaging apparatus 1 is installed at the subject's home etc. Fig. 3B illustrates a flow from installation of the ophthalmohogic imaging apparatus 1 to return, particularly, a mode of usage of the ophthalmologic imaging apparatus 1 at the subject's home etc.

### (S1: Activate an application program for configuration)

First, the configuration regarding the operation of the ophthalmologic imaging apparatus 1 is carried out in a medical institute etc. For this purpose, an application program for configuration is activated. This application program has been installed in the ophthalmologic imaging apparatus 1 or the external computer 1000. As described above, the configuration operation is carried out by using the ophthalmologic imaging apparatus 1 or the external computer 1000. The application program has been installed in the apparatus to be used for the configuration operation.

### (S2: Input setting contents of specific items)

A doctor etc. inputs setting contents of specific items regarding the optical unit 10 and the data processor 130, for example, in the above-mentioned manner.

### (S3: Create setting information)

The main controller 111 creates setting information including the setting contents input in step S2, for example, in the above-mentioned manner.

### (S4: Store setting information)

The main controller 111 stores the setting information created in step S3 in the storage 112, for example, in the above-mentioned manner. The setting information includes, for example, the setting content of the fixation position, the setting content of the scanning pattern, the setting content of the focus position, the setting content of the diopter correction, the setting content of the analysis processing, etc.

### (S5: Install apparatus at subject's home etc.)

The ophthalmologic imaging apparatus 1 is transported to the subject's home etc. after the setting information is stored in step S4. Subsequently, the ophthalmologic imaging apparatus 1 is installed at the subject's home etc. At this time, a fixture for stably installing the ophthalmologic imaging apparatus 1 etc. can be used. Fig. 3B will be referred from the next step.

### (S11: Start examination)

An instruction to initiate the examination is performed. This instruction is, for example, the power-on operation, pushing of an examination initiation button, entry of personal authentication information, etc.

For the case in which personal authentication information is input, for example, the personal authentication information is input in the above-mentioned manner. The authentication processor 134 determines whether or not the input personal authentication information coincides with the authorized personal authentication information stored in the storage 112 in advance. The authentication processor 134 transmits the determination result to the controller 110. If the input personal authentication information coincides with the authorized personal authentication information, the procedure shifts to the examination. On the other hand, if the input personal authentication information does not coincide with the authorized personal authentication information, the main controller 111 outputs a message prompting re-entry of the personal authentication information via the display or by voice. The main controller 111 repeatedly outputs the message until the number of times of determination that they do not coincide with each other reaches a specific number of times (for example, three times). When the determination of discordancy exceeds the specific number of times, the main controller 111 prohibits the examination by the ophthalmologic imaging apparatus 1. Further, the main controller 111 controls the communication part 140, transmitting the information indicating the examination is prohibited (that is, an authentication error is generated) to the external computer 1000. The doctor and/or the person in the manufacturer recognize that the authentication error is generated through the external computer 1000, carrying out specific operations for ending prohibition of the examination (for example, confirmation through a telephone etc.). In addition, the external computer 1000 can be configured to carry out the operation for ending prohibition of the examination. For example, the external computer 1000 transmits the information for personal confirmation to a mobile terminal and/or a computer owned by the subject etc. Password entry is made based on this information. The external computer 1000 determines whether or not a person who has input the password is an authorized subject based on correct or incorrect input of the password. If the person who has input the password is determined to be an authorized subject, the external computer 1000 transmits the information for ending prohibition of the examination to the ophthalmologic imaging apparatus 1. The main controller 111 ends prohibition of the examination based on the information received from the external computer 1000.

### (S12: Is this the first examination?)

When the instruction for initiating the examination has been accepted in step S11, the main controller 111 determines whether or not this examination is the first examination after installation of the ophthalmologic imaging apparatus 1 at the subject's home etc. This processing can be achieved, for example, by adopting the configuration to save the history (log) of the examination in the storage 112 every time the examination is conducted, and by resetting the examination history (examination log) before installation of the apparatus at the subject's home etc. When this examination is determined to be the second or subsequent examination (S12: NO), the procedure shifts to step S14 while skipping step S13. On the other hand, when this examination is determined to be the first examination (S12: YES), the procedure shifts to step S13.

### (S13: Configure respective parts based on setting information)

When this examination is determined to be the first examination in step S12 (S12:YES), the main controller 111 configures settings of the corresponding parts included in the optical unit 10 and the data processor 130 based on the setting information stored in the storage 112 in step S4. This processing includes, for example, any of the following operations: based on the setting content of the fixation position, the display position of the fixation target (this may be a visual target for subjective examination) on the flat panel display 25 is set; based on the setting content of scanning patterns, the control content of the scanner 16 is set; based on the setting content of the focus position, the movement of the objective lens 19 is controlled; based on the setting content of the diopter correction, the movement of the diopter correction lens 27 is controlled; based on the setting content of the maximum interference depth, the movement of the reference mirror 14 is controlled; based on the setting content of the analysis processing, the operational content of the examination data generating part 131 (that is, computer programs to be used) is selected.

Further, as known from the processing flow illustrated in Fig. 3B, the settings configured in step S13 is applied for each round of examination that is conducted until the setting information is changed or abolished in step S23.

In addition, at arbitrary timing after the storage processing of the setting information illustrated in step S4, it is possible to carry out configuration processing based on the setting information. For example, it is possible to carry out the configuration processing before the ophthalmologic imaging apparatus 1 is transported to the subject's home etc. However, in this operational example, the configuration processing is carried out after the apparatus is transported, taking into account the fact that the configuration is accidentally changed. Further, since arrangement of the optical elements is particularly likely to be accidentally changed, other configurations (that is, the configuration of the fixation position, the configuration of the scanning patterns, and the configuration of the analysis processing) may be carried out before transportation of the apparatus.

Moreover, in addition to the setting configuration in step S13, the automatic alignment, the automatic focusing (fine adjustment of the focus position), the automatic tracking, fine adjustment of the maximum interference depth, adjustment of light intensity, and polarization adjustment, etc. may be carried out. The automatic alignment, the automatic focusing, and the automatic tracking are carried out in the above-mentioned manner. The fine adjustment of the maximum interference depth is carried out, for example, by: repeatedly performing line scan; identifying the position of a specific site (fundus surface, retina nerve fiber layer, etc.) of the fundus Ef from moving picture of the cross-sectional images acquired through the repeated line scan; and adjusting the position of the reference mirror 14 such that this specific site is arranged at a specific position in the frame of the moving picture. In addition, the light intensity adjustment is carried out by controlling the attenuator, and the polarization adjustment is carried out by controlling the polarization controller.

### (S14: Start subjective eye examination)

In this operational example, OCT measurement and subjective eye examination are conducted as examinations of the eye E. More specifically, the OCT measurement is conducted in the middle of the subjective eye examination. First, the main controller 111 initiates the subjective eye examination. In other words, the first visual target is presented based on a predetermined optometry program.

In this case, the main controller 111 can display the visual target for the subjective eye examination at the display position of flat panel display 25 based on the setting content of the fixation position included in the setting information. For example, for the case in which a Landolt ring with part of the ring missing is used as the visual target, it is possible to display the Landolt ring such that the missing site is arranged at the position corresponding to the setting content of the fixation position. More generally, it is possible to display, on the flat panel display 25, the visual target such that the part of the visual target to which the subject particularly draws attention is arranged at the position corresponding to the setting content of the fixation position. Thereby, it is possible to provide a fixation function for OCT measurement to a visual target for subjective eye examination.

### (S15: Does the eye remain still?)

Upon initiation of the subjective eye examination, the main controller 111 allows the static-state determination processing of eye E to start. This static-state determination processing is carried out, for example, by the static-state determining part 132 etc. in the above-mentioned manner. The static-state determination processing is carried out at least until the eye E is determined to remain substantially still (S15: NO). If the eye E is determined to remain substantially still (S 15: YES), the procedure shifts to step S16.

When the eye E is not determined to remain still even after the predetermined time has passed from the initiation of the subjective eye examination, or when the eye E is not determined to remain still even after the subjective eye examination has been progressed to a specific stage, it is possible to issue an alert. This alert is issued, for example, by the main controller 111 detecting the advent of the above-mentioned alert timing and controlling the display 210 or the voice output part.

In this operational example, the subjective eye examination is conducted in the middle of the OCT measurement; however, the timing at which these examinations are conducted is optional. For example, the OCT measurement may be conducted after the end of the subjective eye examination, or the subjective eye examination may be conducted after the end of the OCT measurement.

### (S16: Conduct OCT measurement)

In response to the fact that the eye E is determined as remaining substantially still in step S15 (S15: YES), the main controller 111 allows the optical unit 10 to conduct OCT measurement of the eye E. This OCT measurement is conducted with the setting content included in the setting information (for example, the setting content of the fixation position, the setting content of the scanning pattern, the setting content of the focus position, the setting content of the diopter correction).

### (S17: Carry out analysis processing)

The examination data generating part 131 carries out analysis processing based on the data acquired through the OCT measurement. This analysis processing is carried out based on the setting content of the analysis processing included in the setting information. Specifically, fundus-layer-thickness analysis, drusen analysis, disc-shape analysis, etc. are carried out.

### (S18: Subjective eye examination ends)

In the subjective eye examination initiated in step S14, the visual acuity value of the eye E is determined based on the response of the subject to the visual targets sequentially presented in accordance with the optometry program. The subjective eye examination ends when the visual acuity value is determined. Therefore, the end timing of the subjective eye examination is of arbitrary timing after step S14.

### (S19: Generate examination data)

The examination data generating part 131 generates examination data including the data acquired by the analysis processing and the data acquired by the subjective eye examination.

### (S20: Transmit examination data)

The main controller 111 transmits the examination data generated in step S19 to the external computer 1000 by controlling the communication part 140.

Supplementary information such as the examination date and time, the identification information of the subject and/or the eye E, the identification information of the ophthalmologic imaging apparatus 1, etc. may be transmitted together with the examination data. The examination date and time is acquired by the date and time function installed in the main controller 111 (system software). Alternatively, date and time information may be provided from the external computer 1000 etc. Identification information of various types is stored in the storage 112 in advance.

### (S21: Examination ends)

When the examination data is transmitted in step S20, the examination at this time ends. The subject carries out operations such as power-off.

### (S22: Is the apparatus returned?)

The above-mentioned examinations are repeatedly conducted until the ophthalmologic imaging apparatus 1 is returned (S22: NO). The apparatus is returned, for example, due to the end of the examination (follow-up, etc.) at the subject's home etc., replacement of the apparatus, maintenance of the apparatus, change of the setting information, etc. (S22: YES). The ophthalmologic imaging apparatus 1 is transported to the medical institute, the manufacturer, etc. when return of the apparatus is determined.

### (S23: Change/abolish the setting information)

The doctor in the medical institute and/or the person in the manufacturer change or abolish the setting information stored in the ophthalmologic imaging apparatus 1 in step S4. So ends the description of this operational example.

### (Other operational examples)

Other operational examples of the ophthalmologic imaging apparatus 1 will be described mainly with regard to different points from the above-mentioned operational examples.

The subject is instructed to conduct the examinations at a specific time interval (for example, every day, every second day) by the doctor etc. The information indicating this time interval or a longer period of time (collectively referred to as a "specific period of time") is stored in the storage 112 in advance. The main controller 111 monitors intervals of the examinations that are actually conducted at the subject's home etc. This processing is carried out with reference to, for example, the above-mentioned examination history (examination log). For the case in which the examination has not been conducted for the specific period of time, that is, when examination data has not been generated for the specific period of time, the main controller 111 controls the communication part 140 to transmit the annunciation information to the external computer 1000. This annunciation information includes information indicating that the examination has not been conducted for the specific period of time.

There are cases in which examinations of both the right and left eye are conducted at the subject's home etc. In these cases, the setting information includes the left eye setting information and the right eye setting information. In other words, in Fig. 3A, the setting contents regarding both eyes are respectively input in step S2, the setting information including the setting contents of both eyes is generated in step S3, and the setting information including the setting contents of both eyes is stored in the storage 112 in step S4. In addition, the information indicating the eye E is the left eye or the right eye is input from the first input part (the right/left determining part 133 or te manipulator 220) into the main controller 111 in step S11 The main controller 111 selects either the left eye setting information or the right eye setting information based on the input information, and carries out the configuration processing of step S13 based on the selected setting information. In addition, upon end of the examination of left eye or right eye based on the setting information (step S18), the main controller 111 executes control processing for outputting a message (visual information or audio information) for switching the eye subject to the examination. In addition, when a binocular eyepiece to accept both right and left eyes is provided, the main controller 111 controls the optical unit 10, allowing the operation for switching the subject to which visual targets are presented and the subject to which the measuring light is projected to be carried out. This switching operation is carried out in the way in which the destination to which the light flux is guided is switched between the eyepiece for the left eye and the eyepiece for the right eye by driving, for example, a mirror, a polarization element, a wave-selective element, etc. When the examinations of both eyes end, examination data including the examination results of both eyes is generated in step S19, this examination data is transmitted to the external computer 1000 in step S20, and the examinations at this time end (S21).

There are cases in which the ophthalmologic imaging apparatus 1 is shared by two or more subjects. In these cases, the setting information for each subject is stored in the storage 112. In other words, in Fig. 3A, the configurations regarding respective subjects are respectively input in step S2, the setting information of respective subjects is generated in step S3, and then, the setting information of respective subjects is stored in the storage 112 in step S4. In addition, the authorized personal authentication information of respective subjects is stored in the storage 112. The personal authentication information is input in step S11 The main controller 111 searches the authorized personal authentication information coinciding with the input personal authentication information, identifying the setting information corresponding to this authorized personal authentication information. Subsequently, the main controller 111 carries out the configuration processing of step S13 based on the identified setting information.

It is possible to accumulate examination data acquired by the ophthalmologic imaging apparatus 1 in the apparatus. Specifically, the main controller 111 stores the examination data generated in step S19 in the storage 112 at any timing after step S19. Here, it is possible to store the examination date and time in coordination with the examination data. In addition, it is possible to store the information indicating whether the eye E is a left eye or a right eye and the identification information of the subject in coordination with the examination data.

It is possible to inform the fact in which abnormalities in the operational status of the ophthalmologic imaging apparatus 1 are detected. The detection of abnormalities in the operational status is carried out by the monitoring processor 135. A first informing method is carried out via the external computer 1000. In other words, when the monitoring processor 135 detects abnormalities in the operational status, the main controller 111 controls the communication part 140 to transmit information indicating the generation of abnormalities to the external computer 1000. A second informing method involves informing to the subject. In other words, when the monitoring processor 135 detects abnormalities in the operational status, the main controller 111 controls the display 210 and/or the voice output part to output annunciation information (visual information and/or audio information) indicating the generation of abnormalities. The display 210 and the voice output part are examples of the information output part. Such informing processing is carried out at arbitrary timing. When abnormalities are detected during examination, informing processing may be instantly carried out, or informing processing may be carried out after the examination ends. In addition, informing methods and/or informing timings may be differed in accordance with the site at which abnormalities are generated. For example, for the case in which abnormalities of the optical unit 10 are detected, the main controller 111 interrupts the examination if detected during the examination, and instantly informs both the external computer 1000 and the subject. On the other hand, for the case in which abnormalities regarding the analysis processing are detected, the main controller 111 transmits annunciation information to the external computer 1000 after it carries out the processing until the former stage of the analysis processing (OCT measurement, subjective eye examination.)

### [Effects]

The ophthalmologic imaging apparatus 1 is an example of ophthalmologic imaging apparatuses according to embodiments. Hereinafter, the effects of the ophthalmologic imaging apparatuses according to the embodiments will be described.

An ophthalmologic imaging apparatus includes an optical system (for example, the optical unit 10), a processor (for example, the examination data generating part 131), an output part (for example, the display 210 and/or the communication part 140), an interface part (for example, the communication part 140 and/or the user interface 200), a storage (for example, the storage 112), and a controller (for example, the main controller 111).

The optical system is configured to divide light from a light source (for example, the light source 11) into measuring light and reference light, interferes the measuring light having traveled via an eye with the reference light, and detect interference light thereby acquired. The processor generates examination data indicating the status of the eye by processing detection results of the interference light from the optical system. The interface part outputs the examination data generated by the processor. The interface part is used to configure a specific item regarding the optical system and the processor. The storage stores setting information indicating the content of the setting configured via the interface part. For each of multiple examinations that are conducted until the setting information is changed or abolished, the controller controls the optical system and the processor based on the setting content indicated in the setting information.

According to such an ophthalmologic imaging apparatus, once the setting information for defining the operational content of the apparatus is configured, the examination is conducted based on this setting information until this setting information is changed or abolished. Such configuration is different from the conventional ophthalmologic imaging apparatus installed in a medical institute assuming that subject eyes are randomly changed. In addition, it is not necessary to carry out troublesome configuration operations and adjustment operations for each examination since each examination is conducted based on setting information that has been generated in advance. Accordingly, according to the ophthalmologic imaging apparatus of the embodiment, even a person having no knowledge and no experience regarding the apparatus can easily conduct examinations.

The ophthalmologic imaging apparatus according to the embodiment may have not only the above-described OCT measurement function but also the subjective eye examination function. In this case, the optical system includes a flat panel display (for example, the flat panel display 25) that displays a visual target for eye examinations by being controlled by the controller. The optical system presents the visual target to the eye by guiding a light flux output from this flat panel display to the eye. Further, the ophthalmologic imaging apparatus according to the embodiment has a manipulator (for example, the manipulator 220) for inputting responses of the subject with respect to the visual target presented by the optical system. The processor derives a visual acuity value of the eye based on the contents of the response input using the manipulator and generates examination data including this visual acuity value.

This examination data includes one or both of the examination results based on the OCT measurement and the results of subjective eye examination. In other words, when only the OCT measurement is conducted, the former is included in the examination data, while when only the subjective eye examination is conducted, the latter is included in the examination data, and when the OCT measurement and the subjective eye examination are conducted, the former and the latter are included in the examination data.

According to such a configuration, not only morphological data of the eye acquired by the OCT measurement but also functional data acquired by the subjective eye examination can be obtained. Accordingly, it is possible to examine the status of the eye from a broader angle. In addition, such examinations can be conducted at home etc. Accordingly, this makes it possible to carry out medical practices such as medication at appropriate timings.

The setting information may include setting content regarding the fixation position. In this case, the controller can display, on the flat panel display, a fixation target for fixating the eye based on the setting content regarding the fixation position. Thereby, it is possible to conduct the OCT measurement of the desired site of the eye. Further, the fixation target may be a visual target for subjective eye examination or may be a visual target dedicated to fixation.

The controller can control the optical system to detect interference light during the control of displaying a visual target on the flat panel display. In other words, the OCT measurement can be conducted during performing the subjective eye examination. Thereby, it is possible to shorten examination time.

The ophthalmologic imaging apparatus according to the embodiment may have a static-state determining part (for example, the static-state determining part 132) that determines whether or not the eye remains substantially still based on the data optically acquired. In this case, when the static-state determining part determines that the eye remains substantially still, the controller can control the optical system to detect the interference light. In other words, the OCT measurement can be conducted at a timing in which the eye remains substantially still. Thereby, it is possible to prevent a failure in OCT measurement caused by movement of the eye.

The light source can be configured to output infrared light and the flat panel display can be configured to output visible light. In this case, the optical system, which includes a dichroic mirror (for example, the dichroic mirror 18) for composing an optical path of measuring light based on the infrared light from the light source with an optical path of the visible light output from the flat panel display, may be configured to guide the measuring light and the visible light to the eye via this dichroic mirror. According to this configuration, it is possible to perform the OCT measurement and presentation of a visual target by the coaxial optical system.

The output part may include a first communication part (for example, the communication part 140) capable of communicating with a first computer (for example, the external computer 1000) installed in a medical institute via a communication line (for example, the communication line 2000). In this case, the controller can control the first communication part to transmit the examination data generated by the processor and the identification information of the eye to the first computer. According to this configuration, it is possible to transmit the examination data while clearly identifying the eye. Further, the identification information of the eye may be information of a configuration capable of identifying the eye, for example, the identification information provided to the subject, the identification information provided to the eye, the identification information provided to the ophthalmologic imaging apparatus, etc.

The first communication part may be capable of communicating with a second computer (for example, the external computer 1000) installed in a medical institute via a communication line (for example, the communication line 2000). In this case, when examination data has not been generated by the processor for a specific period of time, the controller can control the first communication part to transmit the annunciation information to the second computer. According to this configuration, it is possible to inform the medical institute of the fact that the examination has not been conducted according to plan. Thereby, it is possible to instruct the subject to conduct the examination according to the plan.

For the case in which examinations of both eyes of one subject are conducted using the ophthalmologic imaging apparatus, it is possible to selectively use the setting information of both eyes. For example, the ophthalmologic imaging apparatus according to the embodiment has a first input part (for example, the manipulator 220 or the right/left determining part 133) for inputting information indicating that the eye is a left eye or a right eye into the controller. The left eye setting information regarding the left eye of the subject and the right eye setting information regarding the right eye thereof are stored in the storage as setting information. The controller can select either the left eye setting information or the right eye setting information based on the information input by the first input part, and control the optical system and the processor based on the selected setting information. According to this configuration, it is possible to conduct examination of both eyes using preferable configuration conditions in accordance with each eye.

It is possible to automatically determine which eye is intended for examination, the right eye or the left eye. For example, the first input part may include a right/left determining part (for example, the right/left determining part 133) for determining whether the eye is a left eye or a right eye and inputting the result of this determination into the controller. In this case, the controller selects either the left eye setting information or the right eye setting information based on the determination result input from the right/left determining part, allowing control of the optical system and the processor based on the selected setting information. According to this configuration, it is not necessary to manually configure in order to determine which eye is the subject eye, the right eye or the left eye.

It is possible to provide a function for authenticating the subject. For example, the storage stores the authorized personal authentication information regarding the authorized subject allowed to conduct the examination using the ophthalmologic imaging apparatus in advance. In addition, the ophthalmologic imaging apparatus according to the embodiment has: a second input part (for example, the manipulator 220, the reader, and the biometric authentication information input device) for inputting personal authentication information into the controller; and an authentication processor (for example, the authentication processor 134) for determining whether or not the input personal authentication information coincides with the authorized personal authentication information. When the authentication processor determines that the personal authentication information does not coincide with the authorized personal authentication information, the controller can prohibit the operation of the optical system and the processor. On the other hand, when it is determined that the personal authentication information coincides with the authorized personal authentication information, the examination is conducted by controlling the optical system and the processor based on the setting information. According to this configuration, only the authorized subject can use the present apparatus.

Multiple subjects can share the ophthalmologic imaging apparatus. For example, the authorized personal authentication information and the setting information is stored in the storage in relation to each other for each of two or more authorized subjects. The authentication processor determines whether or not the authorized personal authentication information coinciding with the personal authentication information input by the second input part is stored in the storage. When the authorized personal authentication information coinciding with the input personal authentication information is determined to be stored, the controller controls the optical system and the processor based on the setting information in relation to this authorized personal authentication information. According to this configuration, multiple subjects can share the ophthalmologic imaging apparatus, and it is possible to conduct the examination by precisely using the setting information depending on the subject.

The setting information of the optical system may include the setting content of the scanning pattern. In this case, the optical system includes a scanner (for example, the scanner 16) in order to scan the eye with the measuring light. The controller can control the scanner while conducting the OCT measurement based on the setting content regarding the scanning pattern included in the setting information. According to this configuration, it is possible to conduct the OCT measurement by using a preferable scanning pattern that is configured in advance regarding the eye.

The setting information of the optical system may include the setting content on the focus position in the OCT measurement. In this case, the optical system includes a focus position changing part for changing the focus position of the measuring light. The controller controls the focus position changing part based on the setting content regarding the focus position included in the setting information. According to this configuration, it is possible to conduct the OCT measurement in a preferable focus state that has been stored in advance regarding the eye.

The focus position changing part may have a focusing lens (for example, the objective lens 19 and/or other focusing lens) provided in the optical system and a first driver (for example, the lens-tube driver 19B) that moves this focusing lens along the optical axis. In this case, the setting information includes the information indicating the position of the focusing lens. The controller controls the first driver to arrange the focusing lens at the position indicated in the setting information. Thereby, it is possible to automatically adjust the focus position of the measuring light. In addition, when the objective lens 19 is used as a focusing lens, it is possible to reduce the number of optical elements provided in the optical system, making it possible to simplify the configuration of the optical system.

In addition, the focus position changing part may have a diopter correction lens (for example, the diopter correction lens 27) and a second driver (for example, the lens driver 27A) for inserting or extracting this diopter correction lens into or from the optical system. In this case, the setting information includes the information indicating whether or not a diopter correction lens is used. The controller controls the second driver to insert the diopter correction lens into the optical system when the setting information indicates that the diopter correction lens is used. Thereby, it is possible to automatically perform diopter correction in accordance with the refraction index of the eye.

The setting information of the optical system may include the setting content regarding the maximum interference depth at which the interference intensity between the measuring light and the reference light becomes highest. In this case, the optical system includes a maximum interference depth changing part (for example, the reference mirror 14 and the reference mirror driver 14A) for changing the maximum interference depth. The controller controls the maximum interference depth changing part based on the setting content regarding the maximum interference depth. Thereby, it is possible to automatically adjust the maximum interference depth.

For the case in which the setting information of the optical system includes the setting content of the maximum interference depth, it is possible to apply the following configuration. The optical system includes a beam splitter (for example, the beam splitter 13), a measurement arm, and a reference arm. The beam splitter divides light from a light source into measuring light and reference light. The measurement arm corresponds to a measuring light path that guides the measuring light to the eye, guiding the returned light of the measuring light from the eye to the beam splitter. The reference arm corresponds to a reference optical path including a mirror (for example, the reference mirror 14) for reflecting the reference light to the beam splitter. The optical system detects the interference light of the returned light of the measuring light and the reference light, which is acquired via the beam splitter. The maximum interference depth changing part includes the above-mentioned mirror (for example, the reference mirror 14) and a third driver (for example, the reference mirror driver 14A) that moves the mirror in the optical-axis direction of the reference arm. The setting content of the maximum interference depth includes the information indicating the position of the mirror. The controller controls the third driver to arrange the mirror to the position indicated in the setting information. According to this configuration, it is possible to adjust the maximum interference depth by changing the length of the reference optical path in accordance with the setting content of the maximum interference depth. Further, it is also possible to configure the optical system such that the optical system guides light (one or more lights among light from the light source, measuring light, reference light, and interference light) using optical fibers. In addition, it is possible to provide a mechanism for changing the length of the measuring light path.

The setting information of the processor may include the setting content for carrying out desired analysis processing. In this case, the setting information includes the content of the processing to be carried out by the processor (for example, the examination data generating part 131). For example, the processor may include a layer thickness information generating part (for example, the examination data generating part 131) for generating layer thickness information of a fundus based on the detection results of the interference light from the optical system. For the case in which the content of the processing to be carried out by the processor includes the processing for generating layer thickness information, the controller allows the layer thickness information generating part to generate layer thickness information as the examination data. Thereby, fundus-layer-thickness analysis is performed. Further, the case of executing other analysis processing such as drusen analysis and disc-shape analysis is similar to this. According to this configuration, desired analysis processing can be selectively carried out.

Every time examination data is generated by the processor, the controller can store the examination data in storage (for example, the storage 112) in relation to the date and time information. Thereby, the examination date and time and the examination data (history of examination) can be accumulated in the ophthalmologic imaging apparatus. When this configuration is applied, it is possible to transmit the accumulated data as a whole to an external device (for example, the external computer 1000).

It is possible to remotely monitor the operational status of the ophthalmologic imaging apparatus according to the embodiment. For example, the ophthalmologic imaging apparatus according to the embodiment has a monitoring processor (for example, the monitoring processor 135) for monitoring the operational status of specific site thereof. The output part includes a second communication part (for example, the communication part 140) that can communicate with a third computer (for example, the external computer 1000) via a communication line (for example, the communication line 2000). The controller controls the second communication part to transmit the information indicating the generation of abnormalities to the third computer when the monitoring processor detects abnormalities in the operational status. According to this configuration, it is possible to inform of the generation of abnormalities in the ophthalmologic imaging apparatus via the external computer 1000.

In addition, it is possible to inform the subject and his or her assistant of the generation of abnormalities. For example, the ophthalmologic imaging apparatus according to the embodiment has an information output part (for example, the display 210 and/or the voice output part) for outputting visual information and/or audio information. The controller controls the information output part to output the annunciation information indicating the generation of abnormalities when the monitoring processor detects abnormalities in the operational status. According to this configuration, it is possible to inform the subject etc. of the fact that abnormalities have been generated in the ophthalmologic imaging apparatus, making it possible to stop the examination and inform the person in charge on the outside.

According to the embodiment as described above, it is possible to preferably carry out the ophthalmologic examination at the subject's home etc.

The above-described configuration is merely an example for preferably implementing the present invention. Thereby, various changes (omission, replacement, addition, etc.) may be appropriately made without departing from the scope of the invention

For example, if expenses are caused by use of the ophthalmologic imaging apparatus according to the embodiment, it is possible to apply the configuration for transmitting the information regarding the expenses to the external computer, and/or the configuration for storing this information in the ophthalmologic imaging apparatus.

As an example of the former, the ophthalmologic imaging apparatus transmits the information indicating the type of examination (for example, OCT measurement and analysis processing, and subjective eye examination) together with the patient identification information to a computer installed in the medical institute etc. (a hospital information system (HIS) etc.) via a communication line every time it conducts an examination. This processing is carried out, for example, in such a manner that the main controller 111 creates information to be transmitted and controls the communication part 140 to transmit this information. The medical institute calculates an NHI point regarding the examination conducted by using the ophthalmologic imaging apparatus and the amount of the expenses borne by the patient based on the information received from the ophthalmologic imaging apparatus, creating receipt information for the present patient.

As an example of the latter, every time an examination is conducted, the ophthalmologic imaging apparatus stores the type of this examination (along with the examination date and time etc.). This processing is carried out, for example, in such a manner that the main controller 111 creates information to be stored and stores this information in the storage 112. Further, the ophthalmologic imaging apparatus outputs the stored information at a specific timing. This output timing is, for example, the time when received a request from a computer in the medical institute, the time when the ophthalmologic imaging apparatus is returned, etc. In addition, it may be possible to output the information for a specific appointed day (for example, every Monday, every first Monday, etc.). examples of the method of outputting the information includes a method of outputting the information via a communication line, a method of recording the information in a transportable recording medium (for example, a semiconductor memory), a method of printing the information on a recording paper medium, etc. The medical institute calculates an NHI point regarding the examination conducted by using the ophthalmologic imaging apparatus and the amount of the expenses borne by the patient based on the output information, creating receipt information of the present patient.

Computer programs for realizing the above embodiments can be stored in any kind of recording medium that can be read by a computer. As such a recording medium, for example, a semiconductor memory, an optical disk, a magneto-optic disk (CD-ROM, DVD-RAM, DVD-ROM, MO, and so on), and a magnetic storage (a hard disk, a floppy disk (TM), ZIP, and so on) can be used.

In addition, it is possible to transmit and receive such computer programs via a network such as the internet, LAN, etc.

### [Explanation of Symbols]

- 1: ophthalmologic imaging apparatus
- 10: optical unit
- 10A: unit driver
- 11: light source
- 13: beam splitter
- 14: reference mirror
- 14A: reference mirror driver
- 16: scanner
- 18: dichroic mirror
- 19: objective lens
- 19B: lens-tube driver
- 23: CCD image sensor
- 25: flat panel display
- 27: diopter correction lens
- 27A: lens driver
- 100: computer
- 110: controller
- 111: main controller
- 112: storage
- 120: image forming part
- 130: data processor
- 131: examination data generating part
- 132: static-state determining part
- 133: right/left determining part
- 134: authentication processor
- 135: monitoring processor
- 140: communication part
- 200: user interface
- 210: display
- 220: manipulator
- 1000: external computer
- 2000: communication line

## Claims

1. An ophthalmologic imaging apparatus, comprising:
an optical system configured to divide light from a light source into measuring light and reference light, interfere the measuring light having passed through an eye with the reference light, and detect interference light thereby acquired;
a processor configured to generate examination data indicating the status of the eye by processing the detection results of the interference light via the optical system;
an output part configured to output the examination data generated by the processor;
a controller; and
a manipulator, wherein
the optical system, which comprises a flat panel display configured to display a target for an eye examination by being controlled by the controller, presents the target to the eye by guiding a light flux output from the flat panel display to the eye, and
the processor derives a visual acuity value of the eye based on the content of a response input by using the manipulator when the response of a subject for the presented target is input via the manipulator, generating the examination data including this visual acuity value.

2. The ophthalmologic imaging apparatus according to Claim 1, wherein the controller controls the flat panel display to display a fixation target for fixating the eye.

3. The ophthalmologic imaging apparatus according to Claim 2, wherein the controller, while carrying out control for allowing the flat panel display to display the target, carries out control allowing the optical system to detect the interference light.

4. The ophthalmologic imaging apparatus according to Claim 3, wherein the controller allows the target to be displayed as the fixation target.

5. The ophthalmologic imaging apparatus according to Claim 3, wherein the controller carries out control for allowing the flat panel display to display the fixation target upon carrying out control for allowing the optical system to detect the interference light.

6. The ophthalmologic imaging apparatus according to any one of Claims 1 to 5, comprising a static-state determining part configured to determine whether or not the eye substantially remains still based on the data optically acquired, wherein
the controller carries out control for allowing the optical system to detect the interference light when the static-state determining part determines that the eye remains substantially still.

7. The ophthalmologic imaging apparatus according to any one of Claims 1 to 6, wherein
the light source outputs infrared light,
the flat panel display outputs visible light, and
the optical system, which comprises a dichroic mirror configured to combine an optical path of the measuring light based on the infrared light and an optical path of the visible light output from the flat panel display, guides the measuring light and the visible light to the eye via the dichroic mirror.

8. The ophthalmologic imaging apparatus according to any one of Claims 1 to 7, wherein
the output part comprises a first communication part that can communicate with a first computer placed in a medical institution via communication lines, and
the controller controls the first communication part to transmit the examination data generated by the processor and identification information of the eye to the first computer.

9. The ophthalmologic imaging apparatus according to Claim 8, wherein
the first communication part can communicate with a second computer placed in a medical institution via communication lines, and
the controller controls the first communication part to transmit report information to the second computer when the processor has not generated the examination data for a specific period of time.

10. The ophthalmologic imaging apparatus according to any one of Claims 1 to 9, comprising a first input part configured to input information indicating whether the eye is a left eye or a right eye into the controller, wherein
the controller controls the optical system and the processor based on the information input by the first input part.

11. The ophthalmologic imaging apparatus according to Claim 10, wherein
the first input part comprises a right/left determining part configured to determine whether the eye is a left eye or a right eye, inputting the determination results into the controller, and
the controller controls the optical system and the processor based on the determination results input by the right/left determining part.

12. The ophthalmologic imaging apparatus according to any one of Claims 1 to 11, wherein
the storage stores authorized personal authentication information in advance regarding an authorized subject that is allowed to carry out an examination using this apparatus, and
the ophthalmologic imaging apparatus comprises:
a second input part configured to input personal authentication information into the controller; and
an authentication processor configured to determine whether or not the personal authentication information input by the second input part coincides with the authorized personal authentication information, and wherein
the controller inhibits the operations of the optical system and the processor when the authentication processor determines that the personal authentication information does not coincide with the authorized personal authentication information.

13. The ophthalmologic imaging apparatus according to any one of Claims 1 to 12, wherein the optical system comprises a scanner configured to scan the eye with the measuring light by being controlled by the controller.

14. The ophthalmologic imaging apparatus according to any one of Claims 1 to 13, wherein the optical system comprises a focus position changing part configured to change the focus position of the measuring light for the eye by being controlled by the controller.

15. The ophthalmologic imaging apparatus according to Claim 14, wherein the focus position changing part comprises:
a focusing lens provided in the optical system; and
a first driver configured to move the focusing lens by being controlled by the controller.

16. The ophthalmologic imaging apparatus according to Claim 15, wherein the focusing lens is an objective lens.

17. The ophthalmologic imaging apparatus according to Claim 14, wherein the focus position changing part comprises:
a diopter correction lens; and
a second driver configured to insert or remove the diopter correction lens into or from an optical path of the optical system by being controlled by the controller.

18. The ophthalmologic imaging apparatus according to any one of Claims 1 to 17, wherein the optical system comprises a maximum interference depth changing part configured to change, by being controlled by the controller, the maximum interference depth at which the interference intensity between the measuring light and the reference light becomes maximum.

19. The ophthalmologic imaging apparatus according to Claim 18, wherein
the optical system comprises:
a beam splitter configured to divide the light from the light source into measuring light and reference light;
a measurement arm configured to guide the measuring light to the eye and guide the returned light of the measuring light from the eye to the beam splitter; and
a reference arm comprising a mirror configured to reflect the reference light toward the beam splitter, and wherein
the optical system is configured to detect the interference light between the returned light of the measuring light and the reference light acquired via the beam splitter,
the maximum interference depth changing part comprises the mirror and a third driver configured to move the mirror in the direction along the optical axis of the reference arm, and
the controller moves the mirror by controlling the third driver.

20. The ophthalmologic imaging apparatus according to any one of Claims 1 to 19, wherein
the processor comprises a layer thickness information generating part configured to generate layer thickness information of a fundus based on the detection results of the interference light by the optical system, and
the examination data includes the layer thickness information generated by the layer thickness information generating part.

21. The ophthalmologic imaging apparatus according to any one of Claims 1 to 20, wherein every time examination data is generated by the processor, the controller associates this examination data with date and time information and stores it in the storage.

22. The ophthalmologic imaging apparatus according to any one of Claims 1 to 21, comprising a monitoring processor configured to monitor the operational status of a specific site of the apparatus, wherein
the output part comprises a second communication part that can communicate with a third computer via communication lines, and
the controller controls the second communication part to transmit information indicating the occurrence of abnormalities to the third computer when the monitoring processor detects abnormalities in the operational status.

23. The ophthalmologic imaging apparatus according to Claim 22, comprising an information output part configured to output visual information and/or audio information, wherein
the controller controls the information output part to output report information indicating the occurrence of abnormalities when the monitoring processor detects abnormalities in the operational status.
